# EUROPÄISCHE PATENTSCHRIFT

(11) **EP 3 294 373 B1**
(45) Veröffentlichungstag und Bekanntmachung des Hinweises auf die Patenterteilung: **01.04.2020**
(21) Anmeldenummer: 16721060.8
(22) Anmeldetag: 03.05.2016
(51) Int. Cl.: A61M 5/00, A61M 5/20, A61M 5/31, A61M 5/32

(54) **ANPASSBARE INJEKTIONSVORRICHTUNG**
ADJUSTABLE INJECTION DEVICE
DISPOSITIF D'INJECTION ADAPTABLE

(30) Priorität: 13.05.2015 CH 6672015
(43) Veröffentlichungstag der Anmeldung: 21.03.2018
(73) Patentinhaber: Tecpharma Licensing AG, 3401 Burgdorf (CH)
(72) Erfinder: SCHMID, Lorenz, CH-4500 Solothurn (CH); STETTLER, Peter, CH-3423 Ersigen (CH); KLÖTZLI, Urs, CH-3400 Burgdorf (CH); EICH, Adrian, CH-3374 Wangenried (CH); FRAUCHIGER, Vinzenz, CH-4500 Solothurn (CH)
(86) Internationale Anmeldenummer: PCT/CH2016/000074
(87) Internationale Veröffentlichungsnummer: WO 2016/179713

(56) Entgegenhaltungen:
- EP-A2- 2 335 757
- WO-A1-2011/124633
- WO-A1-2012/127365
- WO-A1-2015/078758
- US-A1- 2004 010 233

## Beschreibung

### 1 Technisches Gebiet

Die Erfindung betrifft Injektionsvorrichtungen, insbesondere Injektionspens und Autoinjektoren, mit welchen ein in einem Produktbehälter enthaltenes fluides Produkt ausschüttbar ist. Bei dem fluiden Produkt handelt es sich insbesondere um ein Medikament. Im Besonderen betrifft die Erfindung eine Injektionsvorrichtung, welche kostengünstig an die Bedürfnisse verschiedener medizinischer Indikationen anpassbar ist.

### 2 Hintergrund

Eine injektionsvorrichtung, wie ein Injektionspen, ein Autopen oder ein Autoinjektor, kann grundsätzlich für die Verabreichung von verschiedensten Medikamenten geeignet sein, sofern das Medikament eine Konsistenz aufweist, welche mit dem Injektionsgerät ausschüttbar ist. Unterschiede ergeben sich jedoch indirekt durch die medizinischen Indikationen, für welche die verschiedenen Medikamente verwendet werden. So können aufgrund der Indikation verschiedene Ansprüche an Grösse, Form, Oberfläche und mechanische Eigenschaften des Gehäuses des Injektionsgerätes stellen. So kann zum Beispiel bei Krankheiten wie multipler Sklerose (MS) die Beweglichkeit des Patienten eingeschränkt sein, so dass an eine Injektionsvorrichtung, welche verwendet wird, um Patienten mit MS zu behandeln, besondere Anforderungen an die Form des Gerätes gestellt werden, damit die Patienten das Injektionsgerät überhaupt handhaben können. So kann es bei medizinischen Indikationen, welche die Handhabung von Injektionsgeräten durch Kinder oder kleinwüchsiger Menschen betreffen, erforderlich sein, dass Injektionsvorrichtungen möglichst schlank und klein ausgestaltet sind.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. "Medikament" kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Aus der US8535274 ist ein injektionsgerät bekannt, bei welchem im Bereich des Handgriffs eine U-förmige Schale aufgeschnappt werden kann. Diese Schale kann ergonomischen wie dekorativen Zwecken dienen und kann von der benutzenden Person einfach ausgetauscht werden. Die U-Form der Schale impliziert, dass das Injektionsgerät nicht vollständig von der Hülse umfasst werden kann, was die dreidimensionale Formgebung einschränkt.

Aus der US2009/0157012 ist eine Spritze mit zusätzlicher Gehäusehülse bekannt, welche schiebbar auf der Spritze gelagert ist. Die manuell durch die benutzende Person in axialer Richtung verschiebbare Hülse ermöglicht einen Sichtschutz der Nadel vor und nach einer Injektion. Die Hülse lässt sich in mehreren Positionen auf der Spritze lösbar verrasten. Die Hülse lässt sich durch die benutzende Person auch vollständig von der Spritze abziehen.

Aus der WO03/105927 ist eine Injektionsvorrichtung bekannt, bei welcher ein Teil der Gehäuseaussenfläche mit einer oberflächenveredelnden Beschichtung versehen werden kann. Diese Beschichtung kann rein dekorativer Natur sein oder auch eine Funktion haben. Nachteilig bei dieser Erfindung ist, dass die dreidimensionale Form der Injektionsvorrichtung nur in kleinstem Masse beeinflusst werden kann.

Aus der WO2011/124633 ist ein Injektionsgerät bekannt, welches auf seiner Mantelfläche mit Differenzierungsschalen 204 und 206 ergänzt werden kann. Diese Schalen können vom trainierten Benutzer am Injektionsgerät zusammengesetzt und auch wieder entfernt werden. Dabei umfassen die Differenzierungsschalen 204 und 206 zueinander komplementär ausgebildete Schnappverbindungselemente, das heisst, die Differenzierungschalen 204 und 206 werden nicht am Gehäuse selbst angebracht, sondern die Schalen sichern sich gegenseitig. Weiter ist das Injektionsgerät so ausgestaltet, dass es nur dann verwendet werden kann, wenn die Differenzierungsschalen 204 und 206 montiert sind. Die Differenzierungschalen 204 und 206 beeinflussen also die Funktionalität des Injektionsgerätes.

Für Hersteller von Injektionsvorrichtungen, Medikamentenhersteller, Krankenversichern und zu versorgende Personen ist es ein Bedürfnis, Injektionsvorrichtungen zur Hand zu haben, welche möglichst günstig, jedoch sicher und wirksam sind. Entsprechend wäre es wünschbar eine Injektionsvorrichtung zu haben, welche ein gleichbleibendes, bewährtes, sicheres und vorgegebenes technisches Design mit einem gleichbleibenden Gehäuse aufweist, wobei über zusätzliche Anbauteile, welche fest und unlösbar mit dem Gehäuse verbunden werden, Form, Haptik, Oberflächentopographie und Ergonomie der Injektionsvorrichtung noch während der Produktion der Vorrichtung angepasst werden können, womit den Bedürfnissen der medizinischen Indikation auf einfache und günstige Weise Rechnung getragen werden kann.

### 3 Aufgabe

Es ist eine Aufgabe der Erfindung eine Injektionsvorrichtung bereitzustellen, welche kostengünstig an die Bedürfnisse verschiedener medizinischer Indikationen anpassbar ist.

### 4 Lösung und allgemeine Beschreibung

Die Aufgabe wird erfindungsgemäss durch den unabhängigen Anspruch 1 gelöst, vorteilhafte Weiterentwicklungen ergeben sich aus den abhängigen Ansprüchen, der Beschreibung und den Figuren.

Die erfindungsgemässe Injektionsvorrichtung weist ein längliches Gehäuse, insbesondere in Form eines länglichen Zylinders, und einen im Gehäuse angeordneten Produktbehälter auf. Bei dem Produktbehälter handelt es sich bevorzugt um eine vorbefüllte Spritze oder eine Karpule. Der Produktbehälter kann im Gehäuse verschiebbar oder fest angeordnet sein. Weiter umfasst die Injektionsvorrichtung einen Ausschüttmechanismus, welcher über manuelle Krafteinwirkung durch die benutzende Person oder durch einen in oder an der Injektionsvorrichtung angeordneten Energiespeicher wie zum Beispiel eine Feder, eine Batterie, ein Gasdruckbehälter, ein Gasgenerator oder eine osmotische Antriebsvorrichtung ein Ausschütten des Produkts bewirken kann. Die Injektionsvorrichtung umfasst weiter, im Falle, dass der Produktbehälter eine Karpule ist, eine lösbar am Gehäuse anbringbare Injektionsnadel, welche einen Flüssigkeitspfad zwischen Karpuleninhalt und Injektionsspitze herstellt. Falls der Produktbehälter eine vorbefüllte Spritze mit festangebrachter Injektionsnadel ist, wird keine separate Injektionsnadel benötigt.

In einem weiteren Aspekt umfasst die Injektionsvorrichtung weiter eine Dosiervorrichtung, welche das Einstellen eine bestimmten Teilmenge oder Dosis des im Produktbehälter vorhandenen Produkt ermöglicht, wobei mit dem Ausschüttmechanismus anschliessend die eingestellte Dosis ausgeschüttet wird. Dabei können Dosiervorrichtung und Ausschüttmechanismus so ausgelegt werden, dass genau eine Dosis ausgeschüttet werden kann oder dass mehrere Dosen hintereinander aus demselben Produktbehälter ausgeschüttet werden können. In einem weiteren Aspekt lassen sich mittels Ausschüttmechanismus mehrere gleichgrosse Dosen ausschütten. Alternativ, wenn die Injektionsvorrichtung als klassischer Autoinjektor ausgeführt ist, wird keine Vorrichtung zu Dosiseinstellung benötigt, da nur eine vorgegebene Dosis verabreicht werden kann.

In einem weiteren Aspekt kann die Injektionsvorrichtung zum Einmalgebrauch bestimmt sein, als dass die Vorrichtung nach der Entleerung des Produktbehälters entsorgt werden muss. In weiteren Ausgestaltungen kann der Produktbehälter ausgetauscht werden und die Injektionsvorrichtung somit wiederverwendbar ausgestaltet sein. Alternative können Teile der Injektionsvorrichtung zusammen mit dem Produktbehälter austauschbar sein, so dass ein sogenanntes semi-disposable System entsteht.

In einem weiteren Aspekt kann die Injektionsvorrichtung eine Nadelschutzhülse umfassen, welche die Injektionsnadel abdeckt. In einer bevorzugten Form ist die Nadelschutzhülse zur Injektion von Produkt in eine Richtung bewegbar, dass sie die Injektionsnadel für die Injektion frei gibt. In einer weiteren bevorzugten Form, bewegt sich die Nadelschutzhülse nach der Injektion wieder über die Injektionsnadel und wird relativ zum Gehäuse fest verriegelt, so dass keine weiteren, insbesondere ungewollte, Stechvorgänge mehr möglich sind.

In einem weiteren Aspekt kann die Injektionsvorrichtung eine Abziehkappe umfassen, welche dasjenige Ende der Injektionsvorrichtung abdeckt, welches die Injektionsnadel beherbergt. Die Abziehkappe kann die Injektionsvorrichtung vor dem geplanten Gebrauch vor ungewollter Auslösung schützen oder die Nadelschutzhülse vor ungewollter Manipulation schützen, zum Beispiel während des Transportes oder dem Auspackvorgang. In einem weiteren Aspekt kann die Abziehkappe dazu verwendet werden um vor dem Gebrauch eine allfällige Nadelschutzkappe von der Injektionsvorrichtung zu entfernen, wie sie zum Beispiel bei Fertigspritzen gebräuchlich ist, um die Nadel insbesondere steril zu halten,

Das Gehäuse der Injektionsvorrichtung ist vorzugweise länglich und bildet die Längsachse der Injektionsvorrichtung. Das Gehäuse ist vorzugsweise hülsenförmig und/oder zylindrisch, insbesondere kreiszylindrisch. Auf der Gehäuseaussenseite sind erfindungsgemäss Befestigungspunkte angeordnet. Bevorzugt liegen sich jeweils zwei Befestigungspunkte auf der Mantelfläche des Gehäuses diametral gegenüber. In bevorzugten Ausführungsformen sind auf der Aussenfläche des Gehäuses zwei bis sechzehn Befestigungspunkte angeordnet, es können jedoch auch mehr oder weniger sein. In vorteilhaften Ausgestaltungen können die Befestigungspunkte sich in axiale Richtung ausdehnen und statt als einzelne Punkte als Befestigungskanäle oder -streifen ausgebildet sein.

In einem weiteren Aspekt umfasst die erfindungsgemässe Injektionsvorrichtung mindestens zwei gehäuseartige Schalen, welche auf der Aussenseite des Gehäuses angebracht werden können, wie im Folgenden ausführlicher erklärt wird. Die gehäuseartigen Schalen umfassen jeweils mindestens eine Haltevorrichtung, welche geometrisch so angeordnet ist, dass sie beim Anbringen der Schale am Gehäuse in einem Befestigungspunkt zu liegen kommt und damit zu Befestigung der Schale am Gehäuse zumindest beiträgt. Bevorzugt umfassen die Schalen mehrere Haltevorrichtungen, um eine sichere Befestigung der Schalen am Gehäuse erzielen zu können. In bevorzugten Formen handelt es sich bei den Haltevorrichtungen um Schnapparme oder Laschen, welche mit den dazu komplementär ausgebildeten Befestigungspunkten auf oder in dem Gehäuse verschnappen, wodurch die Schalen am Gehäuse gehalten werden. Die Schnapparme oder Laschen sind relativ zur Mantelfläche des Gehäuses in einigen Ausführungsformen tangential zum Querschnitt angeordnet, wenn die zugehörige Schale am Gehäuse befestigt ist.

Weiter umfassen die Schalen jeweils mindestens eine Blockiervorrichtung. Die Funktion einer Blockiervorrichtung einer Schale ist, nach dem Befestigen der Schalen am Gehäuse ein Lösen der Haltevorrichtungen einer anderen Schale vom Gehäuse zu verhindern. Bevorzugt und bezugnehmend auf den letzten Absatz ist die mindestens eine Blockiervorrichtung einer Schale so ausgestaltet, dass sie ein insbesondere radiales Auslenken eines Schnapparmes oder einer Lasche einer anderen Schale, so diese am Gehäuse befestigt ist, verhindern kann. Die Schalen werden somit unlösbar (insbesondere formschlüssig) mit dem Gehäuse verbunden. Unlösbar bedeutet, dass die Schalen insbesondere nicht zerstörungsfrei oder ohne Spezialwerkzeug vom Gehäuse getrennt werden können.

In einer ersten bevorzugten und einfach gestalteten Ausführungsform umfasst die Injektionsvorrichtung zwei gehäuseartige Schalen welche identisch sind. Die Schalen sind dabei als Halbschalen ausgebildet. In dieser einfachen Ausführungsform ergänzen sich die beiden Halbschalen zu einem Zylindermantel, bevorzugt kreiszylindrisch, wenn sie am Gehäuse angebracht sind. Im Querschnitt sind die Halbschalen in etwa halbkreisförmig, wobei sich die beiden Halbschalen im Querschnitt zu einem geschlossenen Umfang ergänzen können, bevorzugt einem Kreis. Entlang der offenen Schalenkanten der Mantelfläche sind die Haltevorrichtungen, insbesondere in Form von Schnapparmen, respektive die Blockiervorrichtungen, insbesondere in Rippenform, auf der Innenfläche der Schalen angeordnet, wobei sie durchaus in in etwa tangentialer Richtung über das offene Ende der jeweiligen Schalen herausstehen können. In der vorliegenden Ausführungsform mit zwei identischen Halbschalen liegen sich auf einer Schale eine Blockiervorrichtung und eine Haltevorrichtung auf axial gleicher Höhe gegenüber an den offenen Schalenrändern, also einander diametral gegenüber. Nimmt man bei dieser Anordnung zwei identische Schalen und fügt Sie zu einem Zylinder zusammen, so kommen Haltevorrichtung und Blockiervorrichtung beim Zusammenbau exakt aufeinander zu liegen, wobei in radialer Richtung die Blockiervorrichtung weiter aussen zu liegen kommen muss, um eine Auslenkung der Haltevorrichtung nach aussen zu verhindern. Es kann sinnvoll sein, mehrere Haltevorrichtungen und auch mehrere Blockiervorrichtungen an einer Schale zu haben, um einen sicheren und festen Sitz der Schalen auf dem Gehäuse zu optimieren. Dabei ist es nicht zwingend, dass jeder Haltevorrichtung eine Blockiervorrichtung zugeordnet ist. Für die erfindungsgemässe Ausführung, also der unlösbaren Verbindung zwischen Schalen und Gehäuse, muss lediglich mindestens eine Haltevorrichtung pro Schale durch eine Blockiervorrichtung einer anderen Schale blockiert werden.

Vorteilhaft an dieser einfachen Ausführungsform ist, dass die Halbschalen identisch sind, weshalb die Herstellung der Halbschalen günstiger wird. Werden zum Beispiel die Halbschalen aus Kunststoff im Spritzgussverfahren hergestellt, so wird für die beiden Halbschalen nur ein Werkzeug benötigt, was die Herstellungskosten erheblich reduziert.

Dem Fachmann wird durch die obige Beschreibung direkt klar, dass bei gleichbleibendem Gehäuse der Injektionsvorrichtung die Schalen sehr frei gestaltet werden können, so lange die Position der Befestigungspunkte auf dem Gehäuse berücksichtigt werden und Haltevorrichtungen und Blockiervorrichtungen an den Schalen so angeordnet sind, dass sie zu den Befestigungspunkten, resp. den Haltevorrichtungen passen. Dabei muss nicht jedem auf dem Gehäuse vorhandenen Befestigungspunkt eine Haltevorrichtung zugeordnet werden. Theoretisch würden pro Schale eine Haltevorrichtung sowie eine Blockiervorrichtung reichen, um die zwei Schalen unlösbar mit dem Gehäuse zu verbinden. Bevorzugt werden jedoch mehr Befestigungen, um einen sicheren Sitz der Schalen am Gehäuse zu optimieren. Auch muss nicht jede Befestigung durch eine Blockiervorrichtung unlösbar gemacht werden, pro Schale reicht es, wenn jeweils eine Haltevorrichtung durch eine Blockiervorrichtung einer anderen Schale blockiert wird.

Bei ausladenden Schalengeometrien, das heisst, wenn der Radius einer Schale an der axialen Position des Befestigungspunktes am Gehäuse grösser ist als der Gehäuseradius, werden Haltevorrichtungen respektive Blockiervorrichtungen einfach im Inneren der Schale radial so nach Innen versetzt, dass Haltevorrichtungen respektive Blockiervorrichtungen zu den Befestigungspunkten des Gehäuses passen. Auf diese Weise kann die Form der Injektionsvorrichtung den Bedürfnissen der medizinischen Indikation angepasst werden, ohne die eigentliche Injektionsvorrichtung verändern zu müssen.

In weiteren Ausgestaltungen umfasst die Injektionsvorrichtung zwei gehäuseartige Schalen, welche sich in Form, Material, Farbe und/oder Haptik unterscheiden können. Analog zur Ausgestaltung der ersten Ausführungsform sind an den Halbschalen der weiteren Ausgestaltungen ebenfalls Haltevorrichtungen und Blockiervorrichtungen so angeordnet, dass die Schalen unlösbar am Gehäuse der Injektionsvorrichtung angebracht werden können. Die Tatsache, dass in diesen Ausgestaltungen die Schalen sich unterscheiden, eröffnet weitere Möglichkeiten bei der Anpassung der Injektionsvorrichtung an die Bedürfnisse der medizinischen Indikation. So können asymmetrische, besonders ergonomische Griffe verwirklicht werden, ohne an der eigentlichen Injektionsvorrichtung etwas zu verändern.

In einer Ausgestaltung ist am Gehäuse der Injektionsvorrichtung eine Öffnung vorhanden, welche zum Beispiel den Blick auf den Produktbehälter erlaubt, so kann auch auf einer der Schalen eine Öffnung angeordnet sein, wie zum Beispiel Fenster (mit oder ohne Scheibe), welche grösser, gleich gross oder kleiner als die Öffnung im Gehäuse sein kann und wenn am Gehäuse angebracht, zumindest teilweise den Blick durch die Öffnung der Schale und der Öffnung des Gehäuses auf den Produktbehälter freigibt. Natürlich sind solche Öffnungen auch bei der ersten, oben beschriebenen Ausführungsform denkbar, wobei dann das Gehäuse bevorzugt zwei gegenüberliegende Gehäuseöffnungen aufweist. Ist die Öffnung der Schale kleiner als diejenige des Gehäuses, kann zum Beispiel der Produktbehälter teilbefüllt werden, ohne dass die benutzende Person dies sehen kann, da dann vorteilhaft die Öffnung in der Schale dem Füllstand angepasst ist. Dabei ist eine Änderung des Gehäuses dann unnötig. Vorteilhaft weist die Öffnung in der Schale einen radial nach innen gerichteten Kragen auf, welcher die Sicht auf allfällige Gehäuseteile verdeckt und die Schale mechanisch stabilisiert.

In einer Ausgestaltung erlauben Öffnungen in den gehäuseartigen Schalen Zugriff auf funktionelle Merkmale der Injektionsvorrichtung, wie Knöpfe, insbesondere Auslöseknöpfe und/oder Dosiseinstellknöpfe, Verriegelungen, Anzeigen, Sensoren, Emitter, Empfänger, Nadelschutzhülsenverriegelungen, Dosisanzeigen, Priminganzeigen, End-of-content-Anzeigen, Lichtemittierende Elemente, Akustische Signalgeber, Hautsensoren, Emitter von elektromagnetischer Strahlung, Lichtsensoren, und weitere. Um der bedienenden Person den Zugang zu funktionellen Elementen der Injektionsvorrichtung zu ermöglichen, können die einzelnen gehäuseartigen Schalen mehrteilig aufgebaut sein, insbesondere mit beweglichen Teilen wie Tasten oder Knöpfen versehen sein, welche eine Aktuation durch die bedienende Person an die funktionellen Merkmale am Gehäuse der Injektionsvorrichtung weitergeben und dadurch eine Aktuation dieser funktionellen Merkmale am Gehäuse bewirken. Mehrteilig kann auch bedeuten, dass in bestimmten der gehäuseartigen Schalen Bereiche mit erhöhter Transparenz vorhanden sind, also Sichtfenster, welche den Blick auf bestimmte Gehäusebereiche freigeben. Diese Fenster können als optische Lupe ausgestaltet sein.

Im Gehäuse der Injektionsvorrichtung können Öffnungen vorhanden sein, durch welche Elemente ins Gehäuseinnere eingeführt werden können und in Wechselwirkung mit darin enthaltenen Elementen der Injektionsvorrichtung treten können. Solche Elemente, die ins Gehäuseinnere eingreifen können sind zum Beispiel die weiter oben beschriebenen Knöpfe. Vorstellbar und vorteilhaft können jedoch auch Taster, Kontakte, Schalter, elektrische oder optische Leiter sein.

In gewissen Ausgestaltungen können die Befestigungspunkte von erfindungsgemässen Injektionsvorrichtungen am Gehäuse Bereiche umfassen, welche optisch oder elektrisch leitend sind. Dies um Licht respektive elektrischen Strom ins Gehäuseinnere hinein oder aus dem Gehäuseinneren heraus zu führen. Wobei Licht und elektrischer Strom breit auszulegen sind, so dass zum Beispiel Energie- wie auch Informationstransport oder beides eingeschlossen sein kann. Bei diesen Ausgestaltungen ist es vorteilhaft, wenn die Haltevorrichtungen (und/- oder allenfalls die Blockiervorrichtungen), welche an den leitenden Befestigungspunkten angebracht werden, ebenfalls über optisch oder elektrisch leitende Bereiche verfügen, so dass eine Übertragung von Licht respektive elektrischem Strom oder Strahlung im allgemeinen zwischen gehäuseartigen Schalen und dem Gehäuseinneren möglich ist. So könnten im Gehäuseinneren angeordnete Sensoren und Aktuatoren durch Steuereinheiten, insbesondere elektronische Steuereinheiten, welche ausserhalb des Gehäuses angeordnet sind angesteuert, abgefragt oder abgetastet werden.

Je nach Geometrie der gehäuseartigen Schalen, insbesondere bei ausladenden Geometrien, bildet der Raum zwischen dem Gehäuse und den Schalen Hohlräume, welche vorteilhaft genutzt werden können, so dass weitere interessante Ausgestaltungen einer erfindungsgemässen Injektionsvorrichtung entstehen. So können die Hohlräume zum Beispiel elektronische Baugruppen auf der Innenseite von zumindest einem Teil der gehäuseartigen Schalen angeordnet sein. Diese elektronischen Baugruppen können der Steuerung, Überwachung, Kommunikation oder der Zustandssignalisierung der erfindungsgemässen Injektionsvorrichtung dienen. Weiter können die gehäuseartigen Schalen dieser Ausgestaltungen über elektronische Anzeigeelemente, wie Displays oder einfacher Leuchtelemente, verfügen. Weiter können an gehäuseartigen Schalen dieser Ausgestaltung Antennenelemente angeordnet sein, welche eine drahtlose Informationsübertragung erlauben. Zur Speisung der elektronischen Baugruppen und weiterer Bauteile, kann an den Schalen ein Energiespeicher in Form einer Batterie, eines Akkumulators oder eines Kondensators vorgesehen sein. Sind mehrere Baugruppen an den Schalen angeordneten, so können diese miteinander verbunden und/oder vernetzt sein und Schaltungen bilden.

Zur Kommunikation können die gehäuseartigen Schalen RFID oder NFC Schaltungen enthalten. Auch können die Schalen WLAN oder Bluetooth Module umfassen. Mittels dieser Kommunikationsmittel, kann die Injektionsvorrichtung Informationen drahtlos an andere Geräte übertragen, so wie Messgeräte, Fernsteuerungen, Management Geräte, Smartphones, Computer, oder Computernetzwerke. Umgekehrt kann die Injektionsvorrichtung Informationen und Anweisungen von externen Geräten empfangen.

In einer beispielhaften Ausgestaltung umfasst eine gehäuseartige Schale eine elektronische Schaltung zur Freigabe der Injektionsvorrichtung. Dazu umfasst die Schaltung Kommunikationsmittel in Form eines Bluetooth Moduls sowie einen elektromechanischen Aktuator, welcher einen beweglich an der Schale angeordneten Splint durch eine Öffnung im Gehäuse in das Gehäuse hinein oder aus dem Gehäuse heraus ziehen kann. Der Splint, wenn er in das Gehäuse eingeschoben ist, verhindert ein Auslösen der Injektionsvorrichtung, in dem er den Ausschüttmechanismus relativ zum Gehäuse blockiert. Wird der Splint durch den elektromechanischen Aktuator aus dem Gehäuse herausgezogen, so ist der Ausschüttmechanismus freigegeben und kann von der benutzenden Person zum Ausschütten von Medikament benutzt werden. Interessant an dieser Ausgestaltung ist nun, dass der Befehl zum Herausziehen des Splints via Bluetooth an die Injektionsvorrichtung gesendet werden kann. So kann der Befehl via Internet an ein Smartphone der benutzenden Person übertragen werden. In der Folge reicht eine Smartphone App diesen Befehl via Bluetooth an die Injektionsvorrichtung weiter. Das heisst in Konsequenz, dass zum Beispiel ein betreuender Arzt oder ein Versicherer via Internet die Verabreichung eines Medikamentes freigeben kann. Diese Ausgestaltung kann weiter ausgebaut werden, indem die elektronische Schaltung weiter einen Sensor umfasst, welcher die Auslösung des Ausschüttmechanismus der Injektionsvorrichtung detektieren kann. Vorteilhaft kann der Sensor als Mikrophon ausgeführt sein, welches das charakteristische akustische Muster des Ausschüttmechanismus' erkennen kann. Das Mikrophon hat den Vorteil, dass es nicht durch eine weitere Gehäuseöffnung ins Gehäuse geführt werden muss, sondern einfach in einem Hohlraum zwischen Gehäuse und gehäuseartiger Schale auf der Innenseite der Schale angeordnet sein kann. Sobald das Mikrophon des entsprechende akustische Muster des Ausschüttmechanismus' erkannt hat, generiert die Schaltung eine entsprechende Information, welche via Bluetooth an das Smartphone der benutzenden Person und von dort via Internet an den behandelnden Arzt, den Versicherer oder einen Protokollserver gesandt werden kann. Können mit der Injektionsvorrichtung mehrere Dosen des Medikamentes ausgeschüttet werden, so kann das Mikrophon mehrere akustische Muster erkennen. Eine entsprechende elektronische Schaltung vorausgesetzt, kann das akustische Muster auch dazu genutzt werden, um die Grösse einer Dosis zu bestimmen. Diese erweiterte Ausgestaltung erlaubt sodann eine Complianceüberprüfung durch Arzt oder Versicherer. Die Tatsache, dass die Elektronik der Injektionsvorrichtung ausschliesslich in einer der gehäuseartigen Schalen lokalisiert, ermöglicht es kostengünstig verschiedene Versionen der Injektionsvorrichtung herzustellen. So können an ein und dasselbe Gehäuse gehäuseartige Schalen mit oder ohne Zusatzfunktionen angebracht werden. Im vorliegenden Beispiel könnte in einer Variante der Aktuator mit dem Splint weggelassen werden. Hierdurch wäre der Ausschüttmechanismus nicht mehr blockiert, müsste aber umgekehrt auch nicht mehr via Smartphone freigegeben werden. Der Teil der Injektionsvorrichtung, welcher innerhalb des Gehäuses angeordnet ist, wird durch die Änderung nicht verändert. Und da die gehäuseartigen Schalen die Gehäuseöffnung verdecken, welche für das Einführen des Splints vorgesehen ist, merkt auch die benutzende Person nicht, dass potentielle weitere Funktionen vorhanden wären.

In einer Ausgestaltung umfasst eine der gehäuseartigen Schalen eine elektronische Schaltung zur Schulung der benutzenden Person und einer anschliessenden Freigabe der Injektionsvorrichtung, Die Freigabe kann dabei wie oben beschrieben über einen elektromechanischen Aktuator und einen Splint erfolgen. Die Schaltung umfasst in dieser Ausgestaltung auch einen Lautsprecher zur Ausgabe von Sprache, wobei der Lautsprecher in der gehäuseartigen Schale integriert ist. Die Schaltung umfasst weiter einen nichtflüchtigen Speicher zum Speichern von sprachlichen Anweisungen, zum Beispiel in Form einer MP3 oder AAC Datei, welche durch die Schaltung in ein analoges Signal gewandelt werden kann und als Sprachmitteilung über den Lautsprecher ausgegeben werden kann. Weiter ist die Schaltung mit einem an der gehäuseartigen Schale angeordneten Bedienelement verbunden, zum Beispiel einem Knopf auf der Aussenseite der Schale. Um eine Freigabe der Injektionsvorrichtung zu erreichen, muss die benutzende Person das Bedienelement betätigen, wodurch die Ausgabe der Sprachdatei durch die Schaltung initiiert wird. Die benutzende Person kann sich nun die Sprachdatei, welche Benutzungsanweisungen enthalten kann, anhören. Nach Beendigung der Sprachausgabe, kann die Schaltung über den Aktuator den Splint aus dem Gehäuse herausziehen und damit die Injektionsvorrichtung für eine Benutzung freigeben. In Erweiterung der Ausgestaltung können nach Freigabe, während und/oder nach der Benutzung der Injektionsvorrichtung automatisch oder nach Betätigung des Bedienelementes weitere Sprachdateien ausgegeben werden. So kann in einer gehäuseartigen Schale die ganze Bedienungsanleitung hinterlegt werden und abhängig von den Bedienschritten automatisch an die benutzende Person ausgegeben werden. Bei Injektionsvorrichtungen, welche für mehrere Dosen verwendet werden können, können die Sprachdateien bei jeder oder nur der ersten Benutzung ausgegeben werden. Hier ist es auch denkbar, dass der Splint nach jeder Benutzung wieder ins Gehäuse eingefahren wird und den Ausschüttmechanismus nach jeder erfolgten Benutzung wieder blockiert. Kann die Injektionsvorrichtung nur einmal benutzt werden und die benutzende Person benötigt die Injektionen zum Beispiel täglich, werden typischerweise Sets von mehreren Injektionsvorrichtungen in einer Packung an die benutzende Person abgegeben. Hierbei ermöglicht es der versatile Aufbau der Injektionsvorrichtung, dass ohne grosse Mehrkosten zum Beispiel eine Injektionsvorrichtung mit der Sprachausgabe ausgestattet ist und die restlichen Injektionsvorrichtungen des Sets nicht. Diese Variante lässt sich einfachst durch die Verwendung unterschiedlicher gehäuseartiger Schalen verwirklichen. Zum Beispiel kann die gehäuseartige Schale, welche die elektronische Schaltung enthält eine andere Farbe haben als diejenigen ohne elektronische Schaltung, dass der benutzenden Person klar ersichtlich ist, welches die Injektionsvorrichtung mit der Anleitung ist.

In einer vorteilhaften Ausgestaltung umfasst die Injektionsvorrichtung eine Vorrichtung zur Lebensdauerüberwachung, respektive zur Überwachung des Ablaufdatums des Medikamentes. Eine solche Überwachungsvorrichtung macht für Medikamente Sinn, welche eine beschränkte Haltbarkeit haben und nach einer bestimmten Lagerungsdauer nicht mehr verabreicht werden sollten. Auch kann eine solche Überwachungsvorrichtung eine Temperaturüberwachung beinhalten, welche die benutzende Person alarmiert, wenn die Gefahr besteht, dass das Medikament durch thermischen Impakt in Mitleidenschaft gezogen sein könnte. Der Aufbau und die Funktion der Vorrichtung werden im Folgenden beschrieben. Im vorliegenden, einfach gehaltenen Beispiel sind zwei Befestigungspunkte über einen elektrischen Leiter miteinander verbunden. Die Haltevorrichtungen, welche an den beschriebenen Befestigungspunkten angebracht werden, weisen elektrisch leitfähige Bereiche auf. Diese Bereiche der Haltevorrichtungen kontaktieren den elektrischen Leiter. Die elektrisch leitfähigen Bereiche ihrerseits sind mit elektronischen Baugruppen, die auf der Innenseite einer gehäuseartigen Schale angeordnet sind, leitend verbunden. Die elektronischen Baugruppen bilden eine elektronische Schaltung. Beim Anbringen der Schale am Gehäuse werden die leitfähigen Bereiche an den beiden Haltevorrichtungen durch den Leiter elektrisch verbunden. Hierdurch kann eine weiter an der gehäuseartigen Schale angeordnete Batterie aktiviert werden. Als Folge können die elektronischen Baugruppen mit Energie versorgt werden, wodurch insbesondere ein Timer gestartet wird. Die elektronischen Baugruppen sind weiter mit Anzeigeelementen verbunden, welche auf der Aussenseite der gehäuseartigen Schale sichtbar oder erfühlbar sind. Bei den Anzeigeelementen kann es sich um einfache LEDs handeln, vorstellbar wäre auch eine Display oder ein akustische Signalgeber. Der Timer wird also bei der Endmontage der Injektionsvorrichtung aktiviert. Im vorliegenden Beispiel kann der Timer auf eine bestimmte Zahl Monate eingestellt sein, entsprechend dem Ablaufdatum des Medikamentes. Nach Ablauf des Timers erzeugt die elektronische Schaltung ein Signal, welches den Anzeigeelementen zugeführt wird. Im Falle von LEDs als Anzeigeelemente können diese zu Leuchten beginnen und damit der benutzenden Person signalisieren, dass die Injektionsvorrichtung nicht mehr gebraucht werden soll. Ergänzend kann die elektronische Schaltung mit einem Temperatursensor ausgestattet werden. Mittels dieses Temperatursensors kann die Temperatur innerhalb der Injektionsvorrichtung überwacht werden. Überschreitet die Temperatur innerhalb der Injektionsvorrichtung für eine bestimmte Dauer einen bestimmten Wert, so dass davon ausgegangen werden muss, dass das Medikament in Mitleidenschaft gezogen wurde, dann erzeugt die elektronische Schaltung ein Signal, welches den Anzeigeelement zugeführt wird. Zum Beispiel können dies dieselben LEDs sein, welche auch beim Ablaufdatum zum Einsatz gelangen und damit die benutzende Person von einer tatsächlichen Benutzung warnen. Alternativ können für den Temperaturalarm auch andere Anzeigemittel zum Einsatz gelangen. Ergänzend kann diese Ausgestaltung fakultativ auch einen elektromechanischen Aktuator mit Splint umfassen (siehe auch oben), welcher die Injektionsvorrichtung bei Temperaturalarm blockiert.

Wie beschrieben soll die Verbindung zwischen den gehäuseartigen Schalen und dem Gehäuse bei erfindungsgemässen Injektionsvorrichtung unlösbar sein. Sind an den gehäuseartigen Schalen elektronische Bauteile und allenfalls Energiespeicher angeordnet, so kann es vorteilhaft sein, wenn die gehäuseartigen Schalen über Öffnungen oder Sollbruchstellen verfügen, welche das Entfernen der Schalen zum Beispiel mittels Spezialwerkzeug ermöglichen, um Elektronik und allfällige Energiespeicher sachgerecht entsorgen oder wiederaufbereiten zu können. Dabei ist es vorteilhaft, wenn das Lösen einer Schale vom Gehäuse offensichtliche Spuren auf der Schale hinterlässt, damit klar ersichtlich ist, dass die Schale schon verwendet wurde. Vorteilhaft zerfällt die schale beim Lösen in mehrere Bruchteile. Alternativ könnten beim Lösen auch ein Siegel gebrochen werden oder Löcher auf der Schale entstehen.

In bevorzugten Ausgestaltungen der gehäuseartigen Schalen, sind diese aus Kunststoff hergestellt, insbesondere im Spritzgussverfahren. In vorteilhaften Ausgestaltungen können die gehäuseartigen Schalen sogenannte Zweikomponententeile sein, welche aus zwei Kunststoffen bestehen, die unterschiedliche Eigenschaften aufweisen, zum Beispiel unterschiedliche Härte, was die Haptik der Schalen vorteilhaft beeinflussen kann. Eine Ausgestaltung mit Zweikomponententeile, bei welcher zwei Kunststoffe mit unterschiedlicher Härte zu Einsatz gelangen, kann so gestaltet sein, dass derjenige Bereich der Schalen, welcher der benutzenden Person als Handgriff dient, oberflächlich das weichere Material umfasst, um ein angenehmes und sicheres Grifferlebnis zu erlauben. Die weiche Komponente kann ergänzend oder ausschliesslich auch auf der Innenseite der Schalen zum Einsatz gelangen und dann als mechanisches Dämpfungsmaterial in Bezug auf das Gehäuse zum Einsatz gelangen oder durch potentielle elastische oder plastische Deformierbarkeit/Formanpassung der Aufhebung von Spiel zwischen Gehäuse und Schalen dienen.

In einer weiteren interessanten Ausgestaltung können die gehäuseartigen Schalen schüttgut-, flüssigkeits- oder gelgefüllte Kissen umfassen, welche Ihre Form zum Beispiel der Hand der benutzenden Person anpassen. Als Schüttgut könnten Sand, Hirse, Kleie, oder Kunststoffpartikel zum Einsatz gelangen. Als Flüssigkeiten seien Wasser oder Öle, insbesondere Silikonöle, beispielhaft angeführt. Als Gele seien Biopolymergele, insbesonder Gelatine oder Agar Agar, respektive Gele auf Silikonbasis angeführt.

Die gehäuseartigen Schalen können in weiteren Ausgestaltungen weitere funktionale Merkmale umfassen. So können die Schalen stossdämpfende Elemente, Elemente zur Verhinderung von Fälschungen, insbesondere Hologramme, aufgedruckte Zeichen oder Codes, insbesondere QR-Codes, oder aufgeformte Zeichen oder Codes, insbesondere in Braille-Schrift, umfassen. Weiter können die Schalen temperatursensitive Beschichtungen aufweisen, oder aus temperatursensitiven Kunststoffen aufgebaut sein, welche temperaturabhängig reversibel oder irreversibel ihre Farbe ändern.

Die gehäuseartigen Schalen können auch dekorative Elemente aufweisen, wie Farben und angebaute Formen, zum Beispiel Symbole, Tier- oder Comicfiguren. Diese dekorativen Elemente können dreidimensional von der Schale abragen oder hervorstehen

In einer weiteren Ausführungsform umfasst die Injektionsvorrichtung eine Gruppe von mehr als zwei gehäuseartige Schalen, insbesondere drei oder vier Schalen, welche sich als Ensemble unlösbar am Gehäuse anbringen lassen. Hierbei kann eine Haltevorrrichtung einer ersten Schale durch eine Blockiervorrichtung einer zweiten Schale am Gehäuse blockiert werden. Eine Haltevorrichtung der zweiten Schale kann dann von der Blockiervorrichtung einer dritten Schale am Gehäuse blockiert werden und so weiter. Dabei ist es auch möglich, dass Halte- und Blockiervorrichtungen an den Schalen umgekehrt angeordnet sind und das zwischen zwei Schalen mehrere Halte- und Blockiervorrichtungen zusammengreifen, wenn die Schalen am Gehäuse angebracht sind. Die letzte Schale in einer Gruppe von Schalen für eine Injektionsvorrichtung kann den Umfang um das Gehäuse zuletzt schliessen und mit der ersten Schale zusammengreifen und das Ensemble von Schalen final und unlösbar am Gehäuse befestigen.

Die eigentlichen Haltevorrichtungen an den Schalen sind in vorteilhaften Ausführungsformen der Erfindung als Schnapparme ausgebildet an deren freiem Ende typischerweise eine zahnartige Struktur angeordnet ist, welche in einer dazu komplementären Form an Befestigungspunkten des Gehäuses verrasten kann. Alternativ kann auf den Schnapparmen statt einer zahnartigen Struktur auch eine Öffnung angeordnet sein, welche mit einer zahnartigen Struktur an Befestigungspunkten des Gehäuses verrasten kann. Gemeinsam ist diesen Ausführungsformen, dass zum Lösen der Verrastung eine Auslenkung des Schnapparmes relativ zum Gehäuse notwendig ist. Bei erfindungsgemässen Ausführungsformen wird dieses Auslenken durch die Blockiervorrichtung verhindert, welche an der jeweiligen Schale als Rippe, Balken, Keil oder Vorsprung ausgebildet ist. Dabei ist die genaue Form der Blockiervorrichtung weniger wichtig als ihre örtliche Anordnung an der jeweiligen Schale, welche so gewählt sein muss, dass sie die Lösung der Verrastung verhindern kann.

In einer weiteren Ausführungsform kann beim Anbringen der Schalen auch eine Klebeverbindung zwischen Befestigungspunkt, Haltevorrichtung und/oder Blockiervorrichtung hergestellt werden. Dies ist mit Klebstoffen möglich, welche für die jeweilig verwendeten Werkstoffe geeignet sind. Auch ist es eine mögliche Ausgestaltung, dass es zwischen Haltevorrichtung und Befestigungspunkt keine eigentliche Verrastung gibt, sondern eine reine Klebe-, Löt- oder Schweissverbindung hergestellt wird, welche auf die Blockiervorrichtung ausgeweitet wird. Weiter wären auch Schraub-, Press- oder Nietverbindungen möglich.

Für den Fachmann sind direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben. Die hier und auch im Folgenden aufgeführten Beispiele sollen grundsätzliche Möglichkeiten der Erfindung aufzeigen und sollen keinesfalls einschränkend ausgelegt werden.

### 5 Figuren

Figur 1 : eine Explosionsdarstellung eines Autoinjektors gemäss einer möglichen Ausgestaltung,
Figuren 2a-2c : der Autoinjektor aus Figur 1 in einem Auslieferungszustand, wobei die Figuren 2a bis 2c durch die Längsachse der Vorrichtung verlaufende Schnittansichten sind, wobei die Schnittansichten um die Längsachse winkelversetzt sind,
Figuren 3a-3c : die Vorrichtung und die Ansichten aus den Figuren 2a-2c , wobei sich eine Nadelschutzhülse in ihrer betätigten Position befindet,
Figuren 4a-4c : die Vorrichtung und die Ansichten aus den Figuren 2a-2c , wobei das Vortriebsglied am Ende eines ersten Teilhubs seines Ausschütthubs gezeigt wird,
Figuren 5a-5c : die Vorrichtung und die Ansichten aus den Figuren 2a-2c, wobei ein Vortriebsglied am Ende seines Ausschütthubs gezeigt wird,
Figuren 6a-6c : die Vorrichtung und die Ansichten aus den Figuren 2a-2c , wobei ein Signal, welches das Ende der Produktausschüttung signalisiert, erzeugt wird,
Figuren 7a-7c : die Vorrichtung und die Ansichten aus den Figuren 2a-2c , wobei die Nadelschutzhülse in ihrer Nadelschutzposition ist,
Figuren 8a-8d : perspektivische Ansichten eines mehrteiligen Spritzenhalters gemäss einer ersten Variante,
Figuren 9a-9c : perspektivische Ansichten eines Spritzenhalters gemäss einer zweiten Variante,
Figuren 10a-10d : perspektivische Ansichten eines Spritzenhalters gemäss einer dritten Variante und
Figuren 11a-11c : perspektivische Ansichten eines Spritzenhalters gemäss einer vierten Variante.
Figuren 12a-12c : perspektivische Ansichten eines Spritzenhalters gemäss einer fünften Variante.

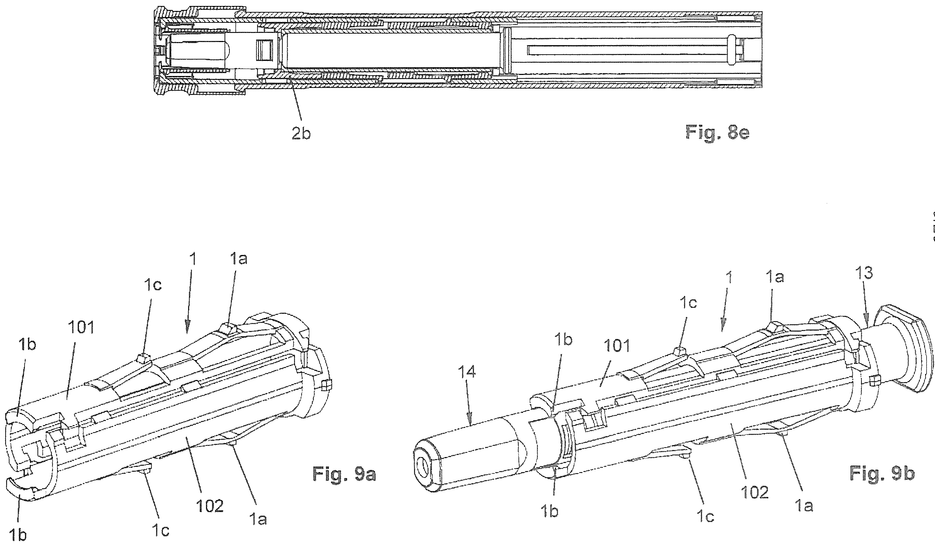

Figuren 8e, 9d, 10e, 11d, 12d : Längsschnitte der fünf Ausgestaltungsformen im Auslieferungszustand und für die Ausgestaltungsformen zwei bis fünf in je einer Position mit teilweise und vollständig eingesetzter Spritze

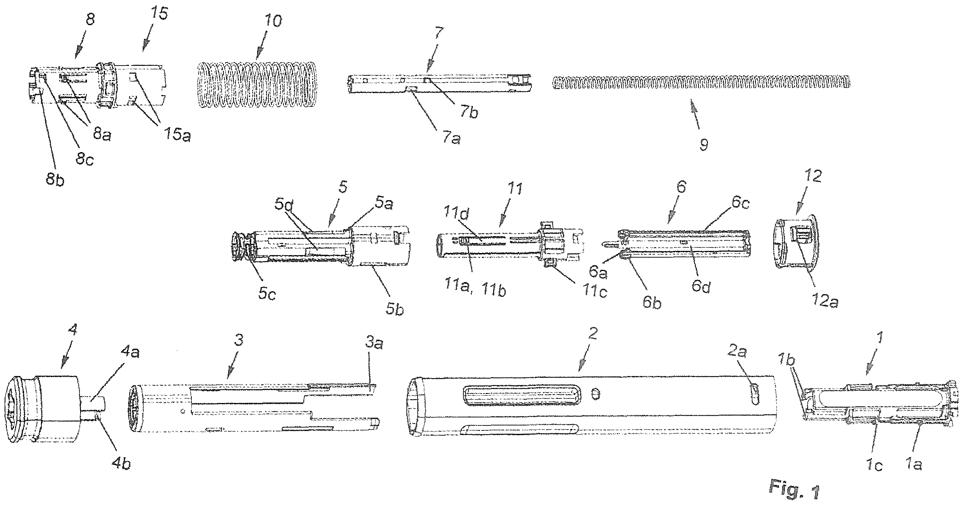

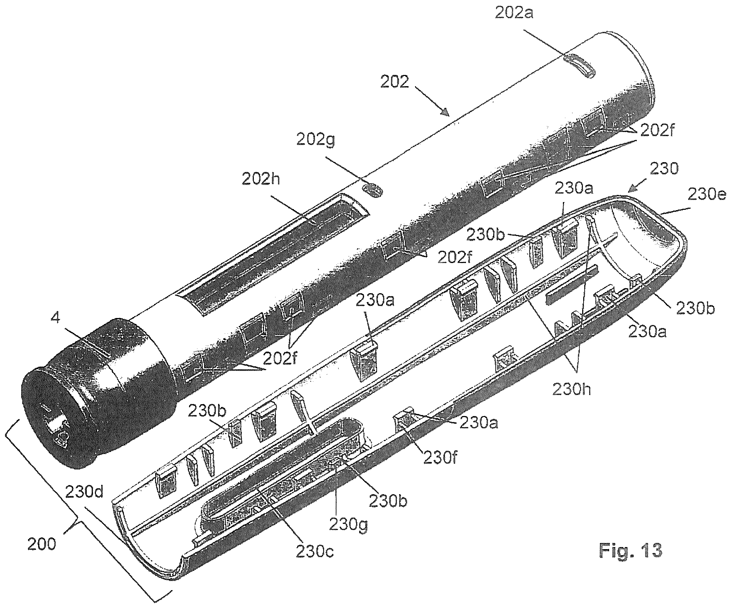

Figur 13 Perspektivische Ansicht eines Autoinjektors mit einer gehäuseartigen Schale gemäss einer erfindungsgemässen Ausführungsform, wobei der Autoinjektor technisch weitgehend dem Autoinjektor aus Figur 1 entspricht

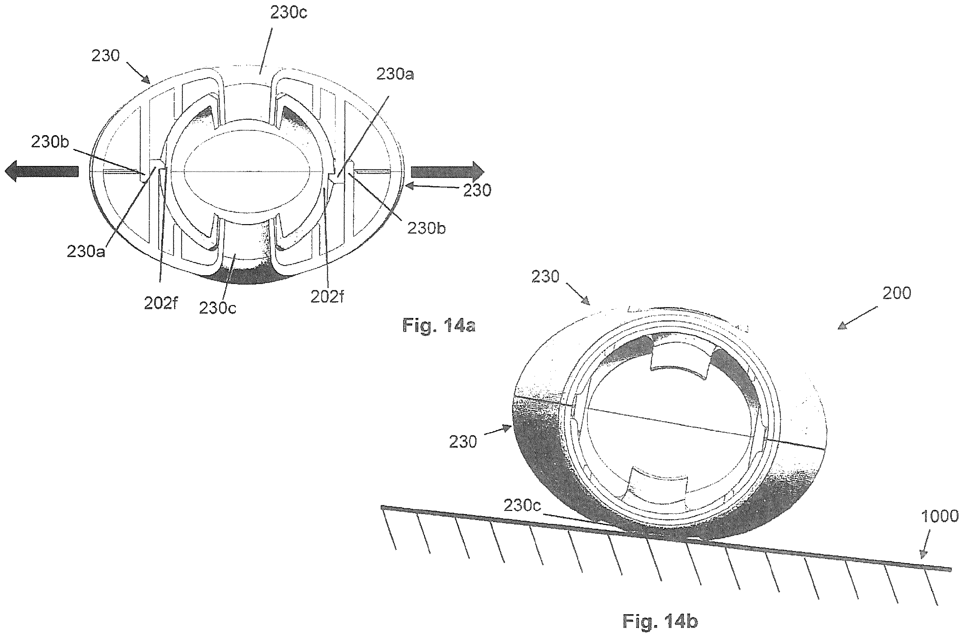

Figuren 14a-14b : Querschnitte durch eine erfindungsgemässe Ausführungsform der Injektionsvorrichtung, wobei
Figur 14b nur die gehäuseartigen Schalen zeigt

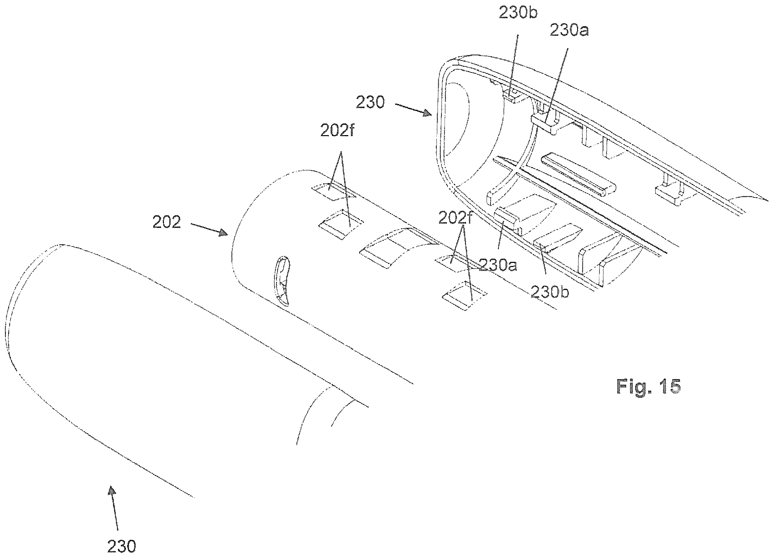

Figur 15 : Perspektivische Detailansicht einer erfindungsgemässen Ausführungsform

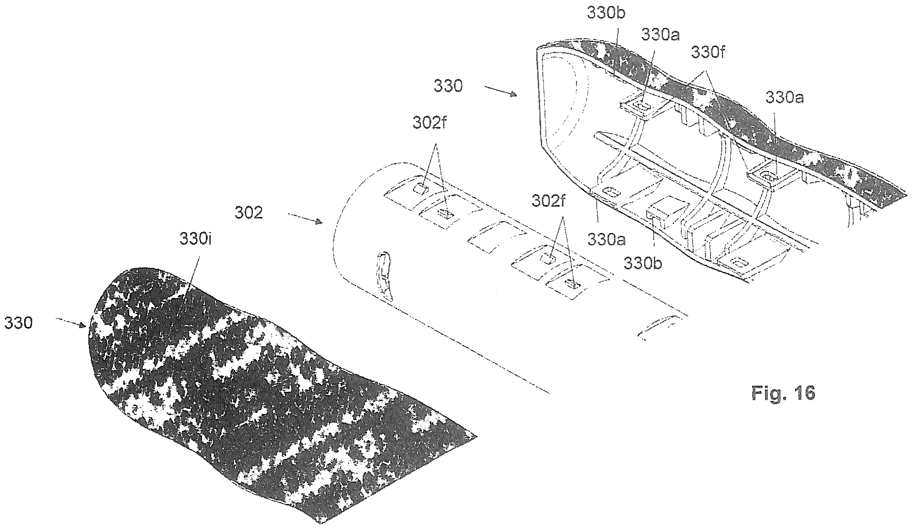

Figur 16 : Perspektivische Detailansicht einer weiteren erfindungsgemässen Ausführungsform

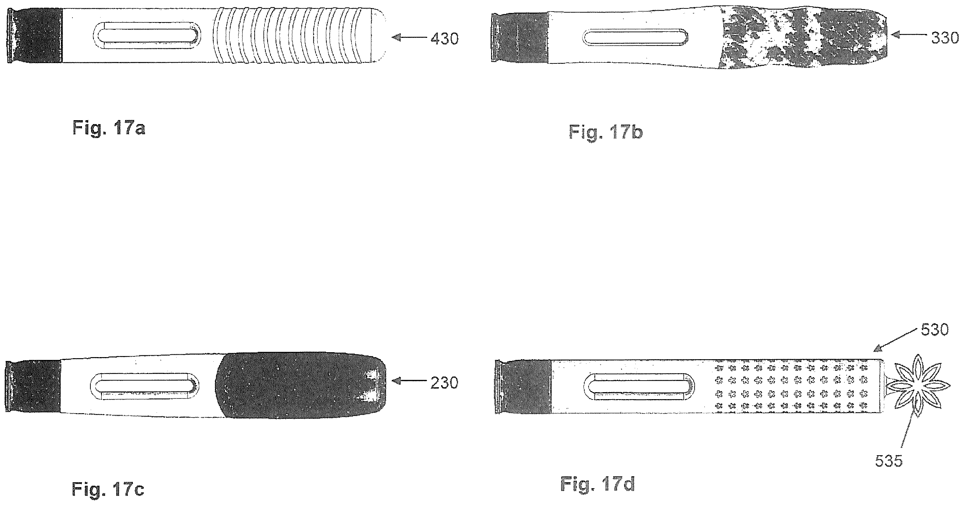

Figuren 17a-17d : Verschiedene mögliche Ausgestaltungen der gehäuseartigen Schalen auf demselben Autoinjektor

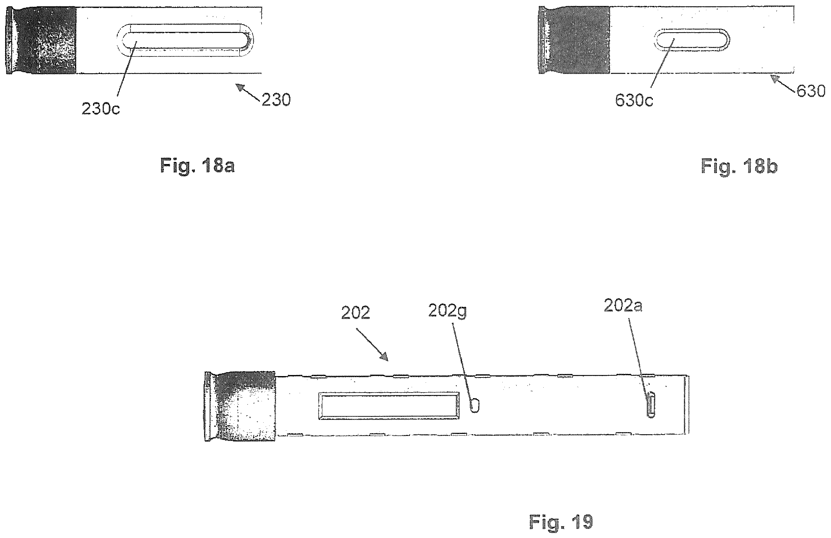

Figuren 18a-18b : Varianten der gehäuseartigen Schalen
Figur 19 : Ansicht des Gehäuses aus Figur 13

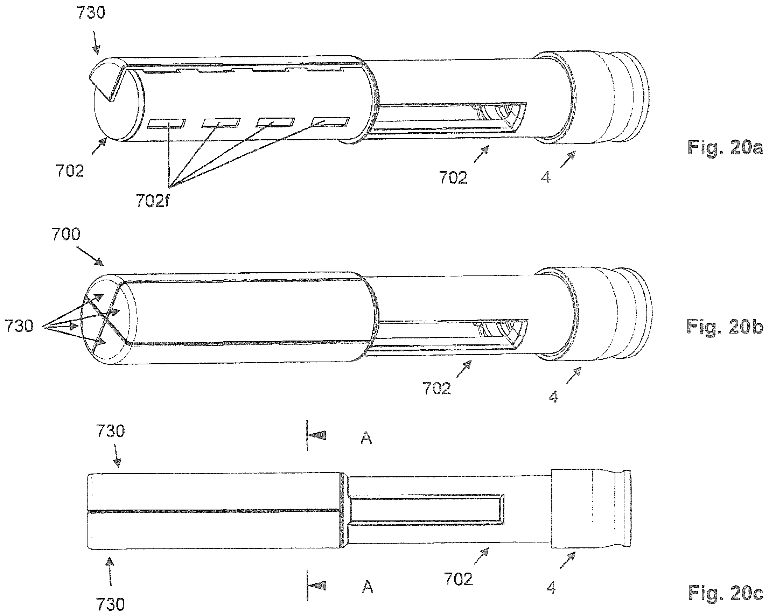

Figuren 20a-20c : Dreidimensionale Ansichten einer erfindungsgemässen Injektionsvorrichtung mit vier gehäuseartigen Schalen

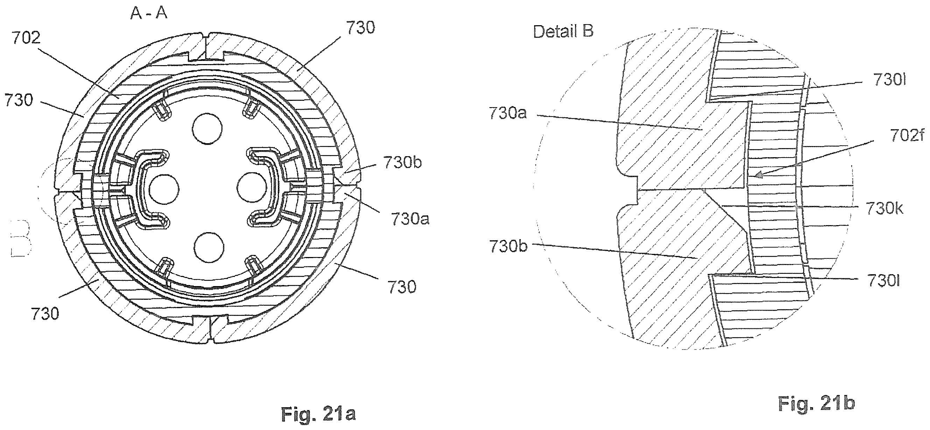

Figuren 21a-21b: Querschnitt und Querschnittdetails der Injektionsvorrichtung aus den Figuren 20a-c

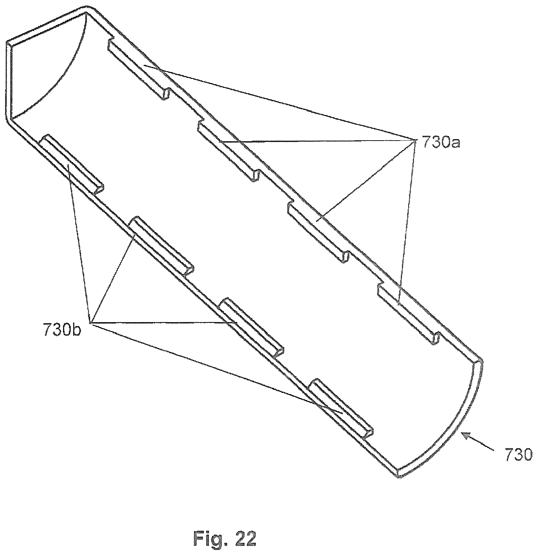

Figur 22: Dreidimensionale Ansicht einer gehäuseartigen Schale aus den Figuren 20a-21b

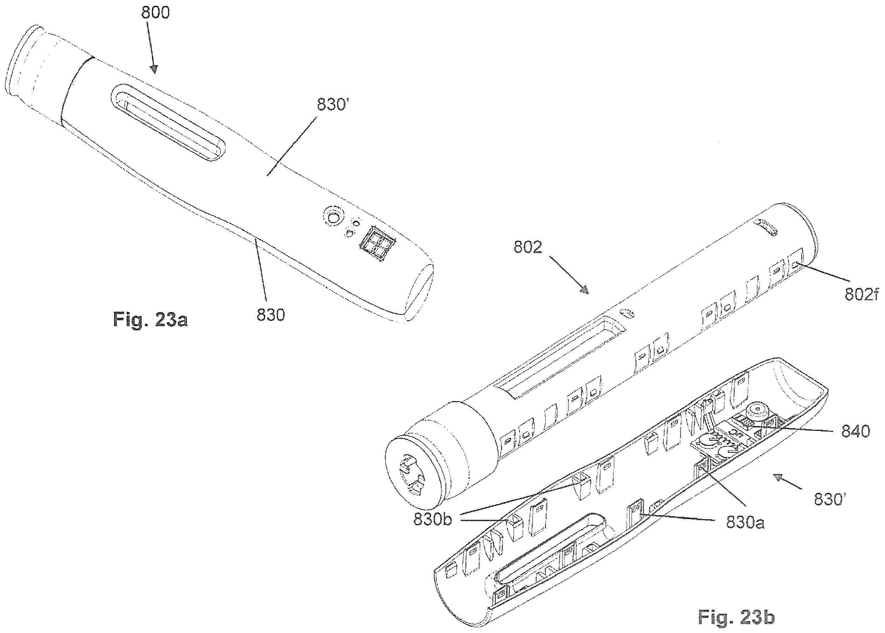

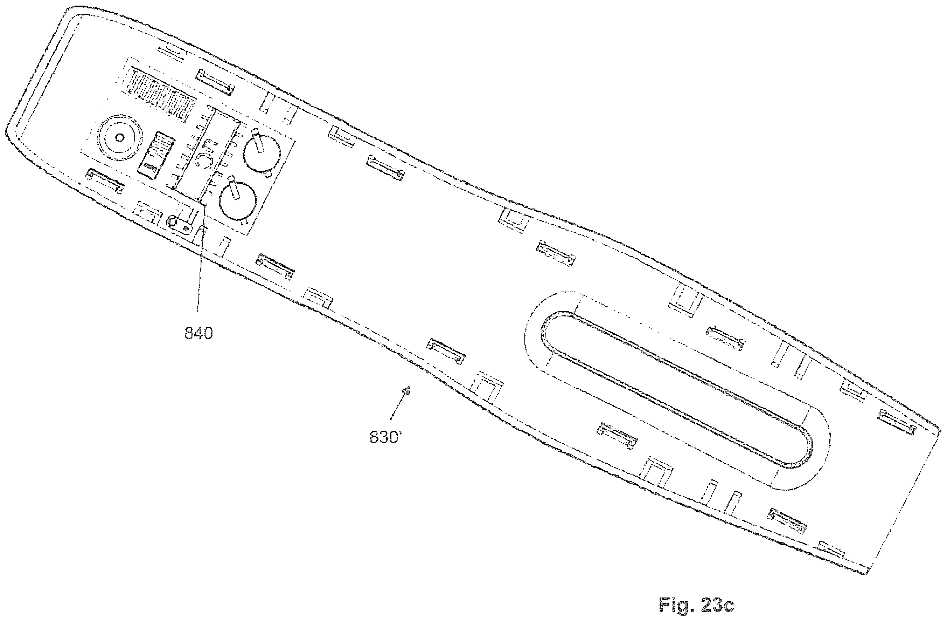

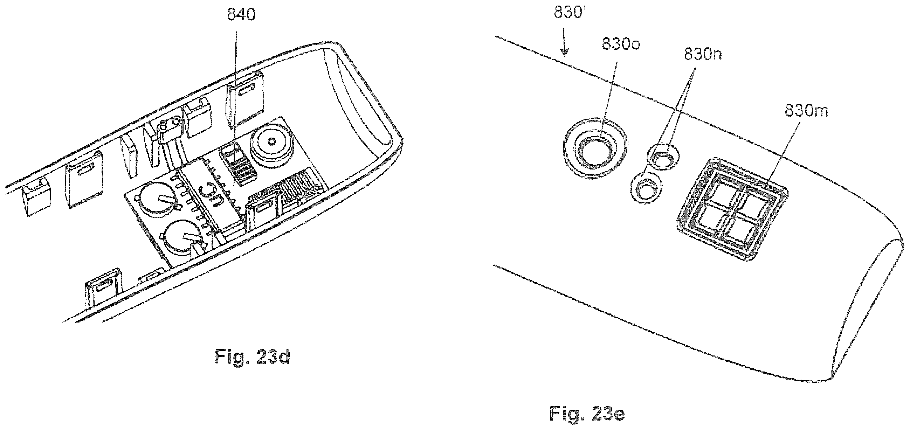

Figuren 23a-23e Gehäuseartige Schale mit Elektronikmodul

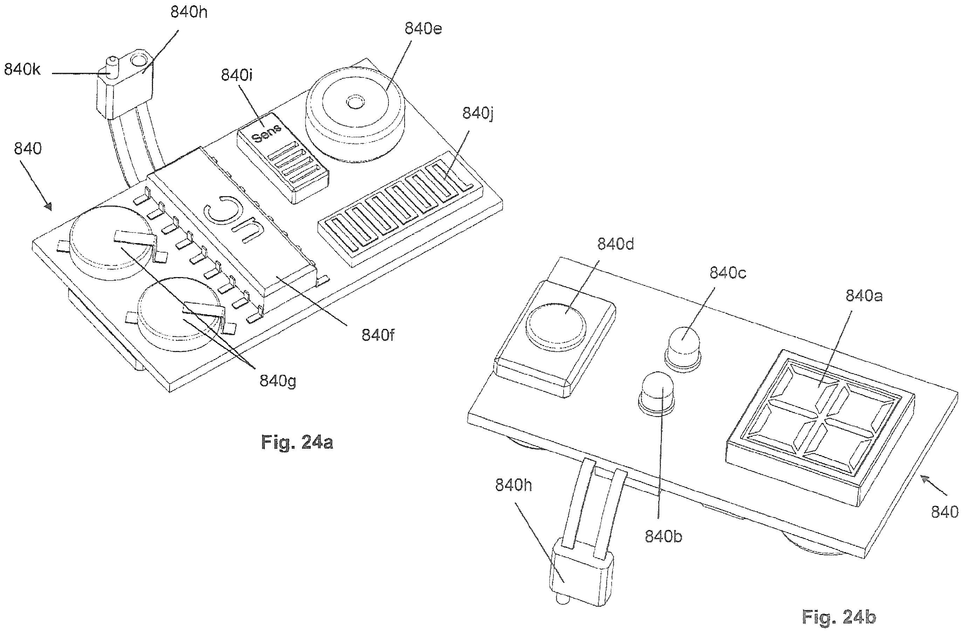

Figuren 24a-24b Detailansichten des Elektronikmoduls

### 6 Figurenbeschreibung

Im Folgenden werden bevorzugte Formen einer Injektionsvorrichtung beschrieben, für welche die Erfindung Anwendung finden könnte, insbesondere Autoinjektoren. Diese Beschreibung soll keinesfalls einschränkend auszulegen sein, sondern lediglich eine mögliche Ausgestaltung aufzeigen. So könnte die Erfindung auch Anwendung an anderen Injektionsvorrichtungen finden, wie zum Beispiel dem ServoPen der Firma Ypsomed oder dem FlexPen der Firma Novo Nordisk.

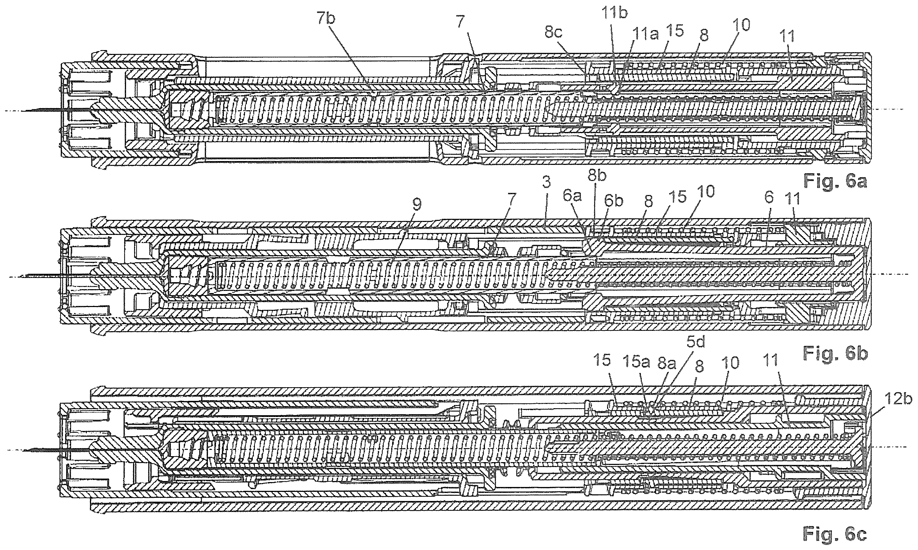

Bezugnehmend auf die Figuren 1-7c werden nun die strukturellen Merkmale und die Funktion des beispielhaften Autoinjektors beschrieben.

Der Autoinjektor, wie er zum Beispiel in Figur 1 gezeigt ist, weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

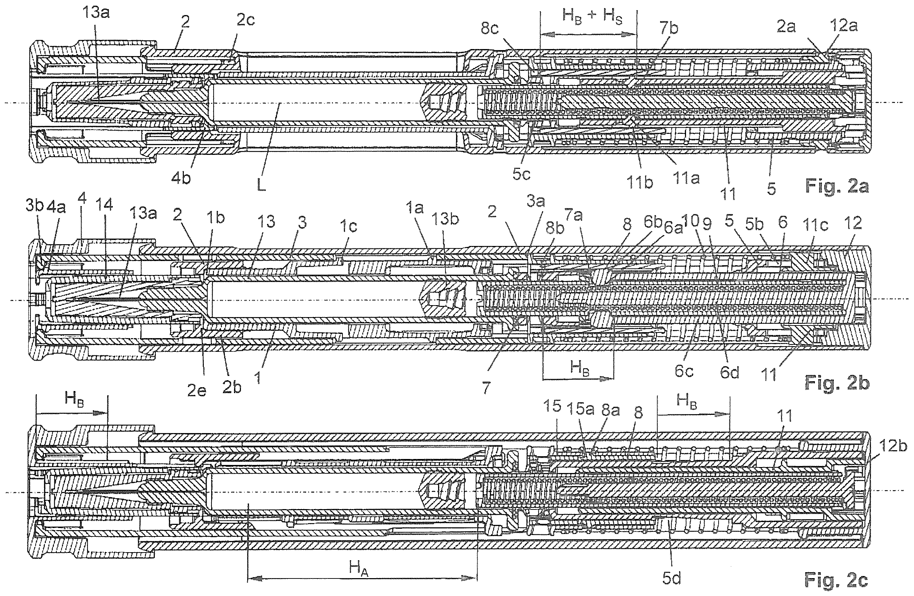

An dem distalen Ende des Autoinjektors ist in seinem Auslieferungszustand (Figuren 2a-2c) eine Abziehkappe 4 angeordnet, die vor der Verwendung des Autoinjektors abgezogen, oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin das Produkt durch die hohle Injektionsnadel 13a aus dem Produktbehälter 13 ausschüttet. Der Spritzenkörper weist an seinem proximalen Ende einen sogenannten Fingerflansch auf, der radial nach aussen über den Aussenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird.

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 2a-2c gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag 2c bilden, an dem die Nadelschutzhülse 3 in der betätigten Position anliegt.

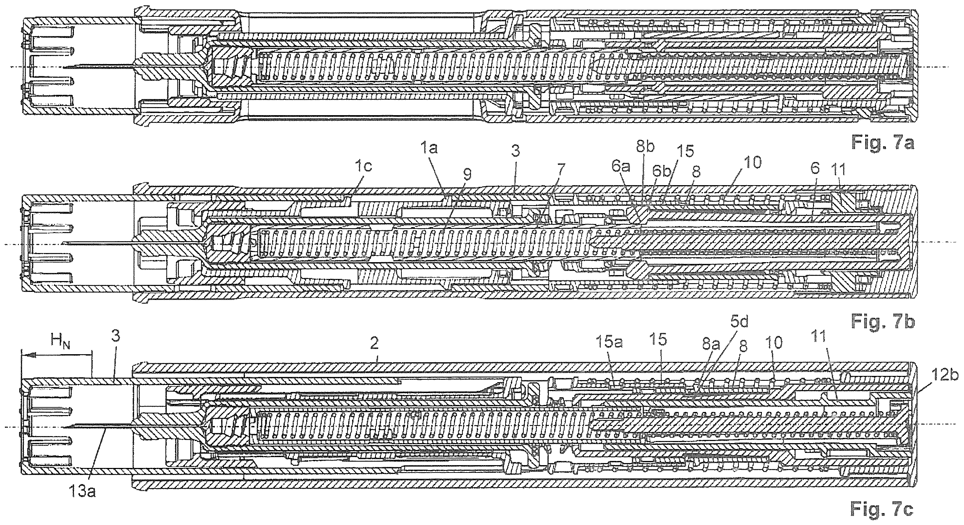

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 7a-7c) verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spitzenhalter 1 weist eine Abragung 1a, die radial nach aussen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2c) und/oder in der Nadelschutzposition der Nadelschutzhülse 3 (Figuren 7a-7c) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 in die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen ringförmigen Halteabschnitt oder Ringabschnitt 2b auf, der insbesondere das distale Ende des Spritzenhalters 1 insbesondere ringförmig umgibt, und daran anliegt, wodurch die mindestens eine Schulter 1b in dem Eingriff mit dem verjüngenden Bereich des Sprilzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhalter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten.

Der Autoinjektor weist ferner ein hülsenförmiges Vortriebsglied 7 auf, welches an seinem distalen Ende eine nach innen ragende Schulter bildet, an der sich eine erste Feder 9, die auch als Ausschüttfeder bezeichnet werden kann, abstützt. Die erste Feder 9 ist innerhalb des hülsenförmigen Vortriebsglieds 7 angeordnet. Die zweite Feder 9 ist eine als Druckfeder wirkende Wendelfeder, die im Ausgangs- oder Auslieferungszustand des Autoinjektors mit so viel Energie vorgespannt ist, dass sie das in dem Produktbehälter 13 enthaltene Produkt insbesondere vollständig durch Verschieben des Vortriebsglieds 7 um einen Ausschütthub H_{A} aus dem Produktbehälter 13 ausschütten kann. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs H_{A} an den Kolben 13b anschlägt und diesen in die Ausschüttrichtung mitnimmt.

Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten. Das Halteelement 6 weist einen Führungsstift 6d auf, der durch das proximale Ende der ersten Feder 9 in die Seele der Feder 9 eingefügt ist. Der Führungsstift 6d verhindert ein seitliches Ausknicken der ersten Feder 9 während und am Ende des Ausschütthubs H_{A} des Vortriebsglieds 7.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der ersten Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, innerhalb der die erste Feder 9 angeordnet ist und die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an einem Signalglied 11, insbesondere an einer Abragung 11c, die axial verschiebbar und verdrehfest in das Gehäuse 2 eingreift und die durch eine schlitzförmige Nut 5b des Mechanikhalters 5 hindurchgreift, ab. Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Das insbesondere hülsenförmige Signalglied 11 ist im Auslieferungszustand oder vor der Auslösung der Produktausschüttung in einem axialfesten Eingriff mit dem Vortriebsglied 7. Das Signalglied 11 weist ein erstes Eingriffsglied 11a, welches in eine Ausnehmung 7b des Vortriebsglieds 7 eingreift, und ein zweites Eingriffsglied 11b auf. Das erste Eingriffsglied 11a und das zweite Eingriffsglied 11b sind an dem Ende eines Arms 11d federnd angeordnet. Das Signalglied 11 weist zwei solcher Arme 11d mit einem ersten Eingriffsglied 11a und einem zweiten Eingriffsglied 11b auf. Das erste Eingriffsglied 11a weist radial zu der Längsachse L hin, wobei das zweite Eingriffsglied 11b radial von der Längsachse L weg weist. Im Auslieferungszustand wird das erste Eingriffsglied 11 a von dem Innenumfang der Sperrhülse 8 in dem axialfesten Eingriff mit dem Vortriebsglied 7 gehalten. Das zweite Eingriffsglied 11b liegt an dem Innenumfang der Schalthülse 8 an. Die Verschlusskappe 12 weist einen Signalanschlag 12b auf, an den das Signalglied 11 zur Erzeugung eines Signals anschlagen kann und vorzugsweise an dem das Signalglied 11 im Auslieferungszustand der Vorrichtung anliegt.

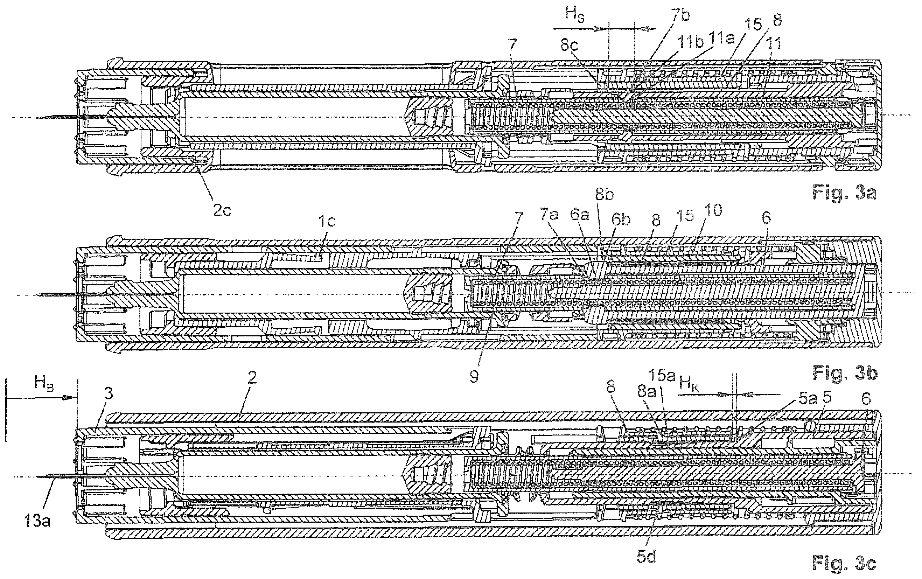

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine erste Ausnehmung 8b auf, die durch Verschieben der Sperrhülse 8 um den Betätigungshub H_{B} entlang der Längsachse L auf die Position des zweiten Eingriffselements 6b gebracht wird, wie in den Figuren 3a-3c dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren erster Ausnehmung 8b bewegt wird. Hierdurch wird das Vortriebsglied 7 für die Bewegung um den Ausschütthub H_{A} in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der ersten Feder 9 in die proximale Richtung bewegt werden, wobei das Halteelement 6 über den Eingriff des zweiten Eingriffselements 6b in die Ausnehmung 8b die Sperrhülse 8 um einen Startsignalhub H_{K} (Figur 3c) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist.

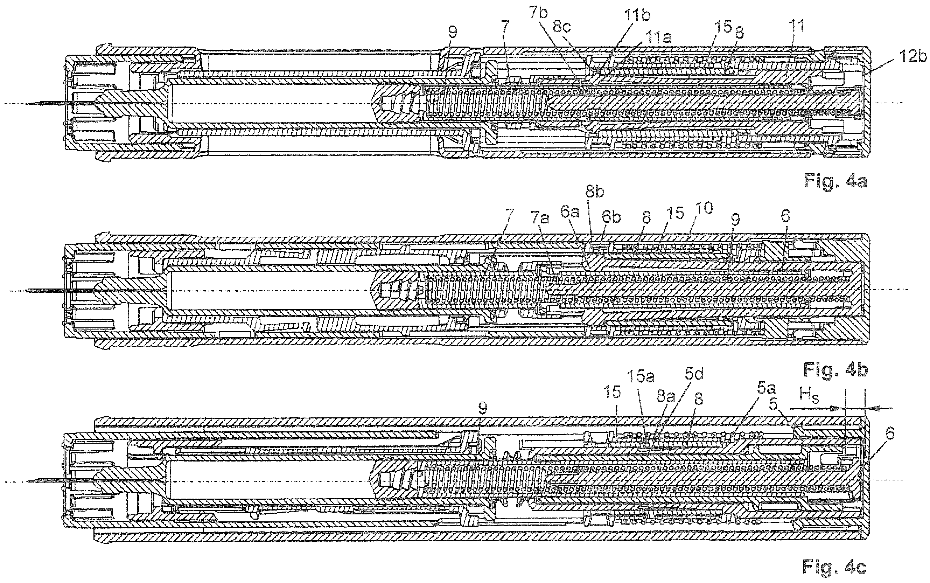

Da das Signalglied 11 noch axialfest mit dem Vortriebsglied 7 verbunden ist, wird es um einen ersten Teilhub Hs des Ausschütthubs H_{A} in die Ausschüttrichtung mitgenommen, wobei das Signalglied 11 in etwa um den ersten Teilhub Hs von dem Signalanschlag 12b weg bewegt wird, wie am besten aus Figur 4c erkennbar ist. Am Ende des ersten Teilhubs H_{S}, während dem das erste und zweite Eingriffsglied 11a, 11b relativ zu der Sperrhülse 8 bewegt werden, wird das erste Eingriffsglied 11a aus dem Eingriff mit dem Vortriebsglied 7 gedrückt, wobei gleichzeitig das zweite Eingriffsglied 11b in die zweite Ausnehmung 8c der Sperrhülse 8 mit einer Bewegung quer zu der Längsachse L und radial von der Längsachse L weg bewegt wird. Hierdurch wird das Signalglied 11 daran gehindert, sich in die proximale Richtung relativ zu dem Gehäuse 2 oder der Sperrhülse 8 zu bewegen. Das zweite Eingriffsglied 11b wird von dem Aussenumfang des Vortriebsglieds 7 in den Eingriff mit der Ausnehmung 8c gehalten (Figur 4a), wenn das Vortriebsglied 7 um seinen zweiten Teilhub des Ausschütthubs H_{A} bewegt wird. Die Aussenumfangsfläche des Vortriebsglieds 7 hält das zweite Eingriffselement 6b in dem Eingriff mit der ersten Ausnehmung 8b der Sperrhülse 8, wie am besten aus Figur 4b erkennbar ist. Am Ende des Ausschütthubs H_{A} gibt das Vortriebsglied 7 das zweite Eingriffsglied 11b aus dem Eingriff mit der Sperrhülse 8 frei, wodurch das zweite Eingriffsglied 11b aus dem Eingriff mit der Ausnehmung 8c bewegt wird, insbesondere zu der Längsachse L hin, so dass die zweite Feder 10 das Signalglied 11 entgegen die Ausschüttrichtung, d. h. in die proximale Richtung beschleunigt, so dass beim Auftreffen des Signalglieds 11 auf dem Signalanschlag 12b ein akustisches und/oder taktiles Signal erzeugt wird.

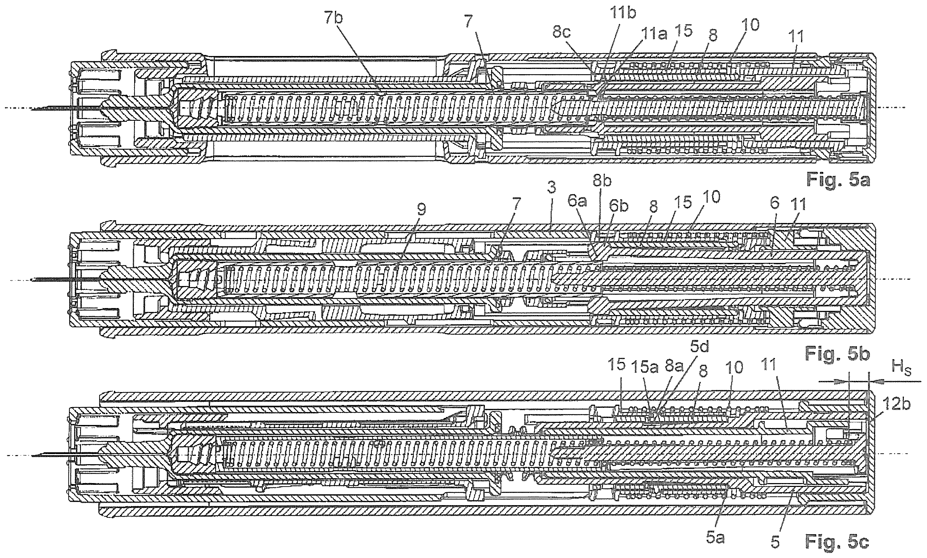

Wie am besten aus Figur 5b erkennbar ist, bleibt der Eingriff des zweiten Eingriffselements 6b in die erste Ausnehmung 8b bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen, wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stösst das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

Im Folgenden werden verschiedene Ausführungen eines Spritzenhalters gezeigt, die bei einem Autoinjektor, vorzugsweise aber nicht notwendigerweise einem Autoinjektor der oben beschriebenen Art, eingesetzt werden können.

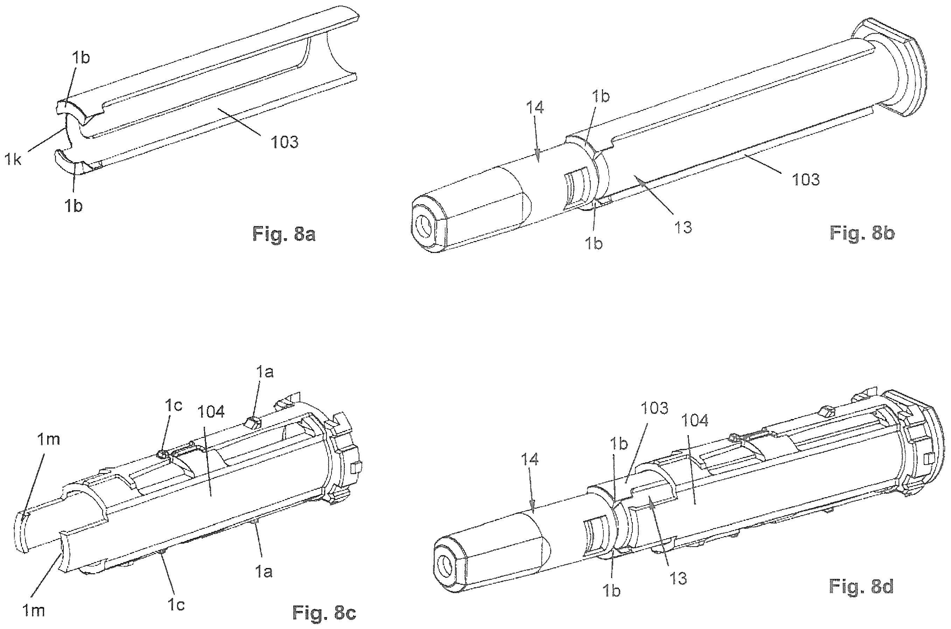

Das Spritzenmodul aus den Figuren 8a bis 8d weist einen ersten Schalenkörper oder Hülsenkörper 103 auf, der seitlich eine Öffnung aufweist und mindestens eine, d. h. in dem gezeigten Beispiel zwei schulterförmige Eingriffsglieder 1b aufweist, die nach innen, d. h. zur Längsachse des Hülsenkörpers 103 ragen. Ferner weist der Hülsenkörper 103 einen in die distale Richtung weisenden Translationsgegenanschlag 1k auf. Für die Montage der Spritze 13 (Figur 8b) wird diese seitlich, d. h. mit einer Bewegung quer zur Längsachse in den Hülsenkörper 103 eingefügt, wodurch das mindestens eine Eingriffsglied 1b in die Lücke zwischen der Nadelschutzkappe 14 und dem sich verjüngenden Abschnitt des Spritzenkörpers der Spritze 13 eingefügt werden.

Das Spritzenmodul weist ferner einen zweiten Schalenkörper, insbesondere Hülsenkörper 104 auf (Figur 8c), der an seinem proximalen Ende offen ist und an seinem distalen Ende mindestens einen, d. h. in dem gezeigten Beispiel zwei Translationsanschläge 1m aufweist, die radial nach innen abragen. Wie in der Ausführung aus den Figuren 1 bis 7c weist der Hülsenkörper 104 mindestens einen Nocken 1c, nämlich zwei Nocken 1c und mindestens eine Abragung 1a, nämlich zwei Abragungen 1a auf. Der Nocken 1c ist über einen Arm federnd an dem Hülsenkörper 104 angeordnet.

Die Einheit aus Spritze 13, Nadelschutzkappe 14 und dem ersten Hülsenkörper 103 wird über das proximale Ende entlang der Längsachse mit der Nadelschutzkappe 14 voran (Figur 8b) in den zweiten Hülsenkörper 104 (Figur 8c) eingeschoben, wobei der Translationsgegenanschlag 1k an den Translationsanschlag 1m anschlägt, wenn die Einheit 13, 14, 103 vollständig in den Hülsenkörper 104 eingeschoben ist (Figur 8d). Die in Figur 8d gezeigte Einheit wird dann für die Montage so in dem Gehäuse 2 des Autoinjektors verschoben, dass der Halteabschnitt 2b, insbesondere der ringförmige Halteabschnitt oder Ringabschnitt, an zumindest dem ersten Hülsenkörper 103 zumindest im Bereich des Eingriffsglieds 1 b anliegt, so dass das Eingriffsglied 1b in dem Eingriff mit dem sich verjüngenden Abschnitt des Spritzenkörpers gehalten wird. Ferner kann der Halteabschnitt 2b auch an dem zweiten Hülsenkörper 104 anliegen, insbesondere in dem Bereich, an dem der mindestens eine Translationsanschlag 1m gebildet ist, um den Translationsanschlag 1m im Eingriff mit dem Translationsgegenanschlag 1k zu halten.

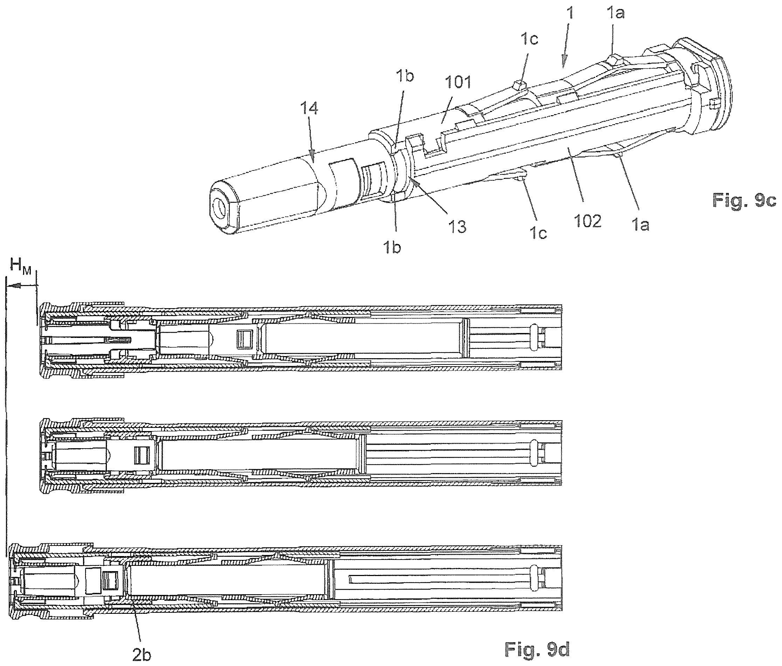

In der in den Figuren 9a-9c gezeigten Ausgestaltungsform weist das Spritzenmodul, insbesondere der Spritzenhalter einen ersten Schalenkörper 101 und einen zweiten Schalenkörper 102 auf, die jeweils als Halbschale gebildet sind. Jeder der Schalenkörper 101, 102 weist einen Nocken 1c und eine Abragung 1a in der hierin beschrieben Weise auf.

Der erste Schalenkörper 101 und der zweite Schalenkörper 102 sind in der in Figur 9a gezeigten Ansicht über mehrere Sollbruchstellen einteilig miteinander verbunden, wobei der erste und zweite Schalenkörper 101, 102 eine Einfügeposition zueinander einnehmen. Über das proximale Ende des in Figur 9a gezeigten Körpers 101, 102 wird die Spritze 13 mit der Nadelschutzkappe 14 voran in die distale Richtung eingeschoben (Figur 9b) bis die Lücke zwischen dem sich verjüngenden Abschnitt und der Nadelschutzkappe 14 entlang der Längsachse L auf der gleichen Position angeordnet ist wie das mindestens eine Eingriffsglied 1b. In dem gezeigten Beispiel weist jeder aus erstem und zweitem Schalenkörper 101, 102 ein Eingriffsglied 1b auf. Durch Gegeneinanderdrücken der ersten und zweiten Schalenkörper 101,102 quer zur Längsachse L werden die Sollbruchstellen zerbrochen, wobei der erste und zweite Schalenkörper 101, 102 formschlüssig miteinander verrasten und die Eingriffsglieder 1b in die Lücke bewegt werden. Wie bereits beschrieben können der Bereich des ersten und zweiten Schalenkörpers 101, 102, an dem das Eingriffsglied 1b gebildet ist, von dem Halteabschnitt 2b des Gehäuses 2 eingefasst werden, wodurch die Eingriffsglieder 1b in dem Eingriff mit dem sich verjüngenden Bereich des Spritzenkörpers gehalten werden. Besonders bevorzugt können sich die Schalenkörper 101, 102 beim Einschieben der Spritze 13 quer zur Längsachse gegen die elastische Kraft der Arme welche die Abragung 1a und/oder den Nocken 1c tragen in die Einfügeposition bewegen. Wie beschrieben können anschliessend auch hier die Eingriffsglieder 1 b von dem Halteabschnitt 2b des Gehäuses 2 in Eingriff mit dem sich verjüngenden Abschnitt des Spritzenkörpers 13 gebracht und gehalten werden. Alternativ oder zusätzlich können der erste Schalenkörper 101 und der zweite Schalenkörper 102 in der Schliessposition (Figur 9c), in der die Eingriffsglieder 1b in die Lücke eingreifen, miteinander verrasten.

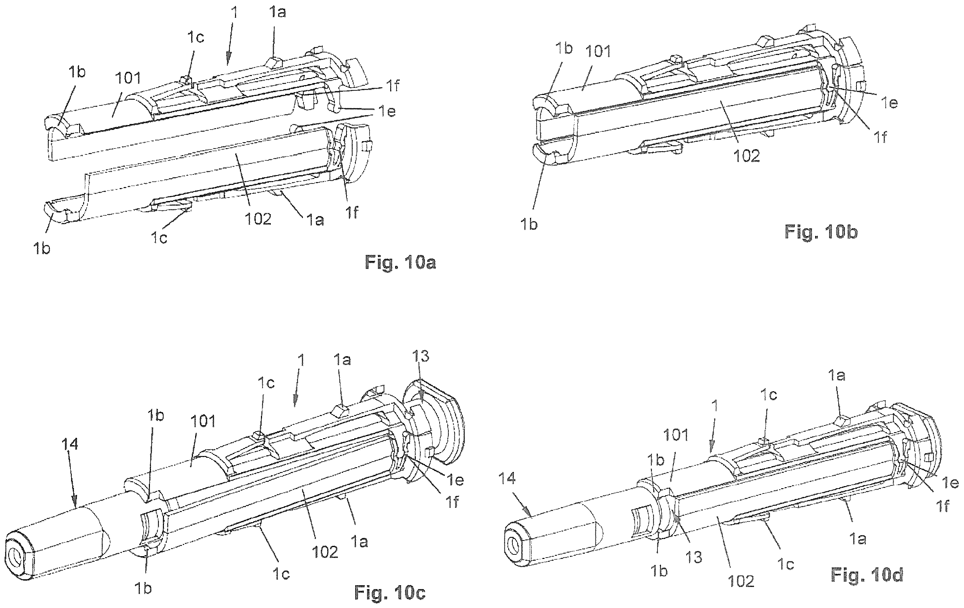

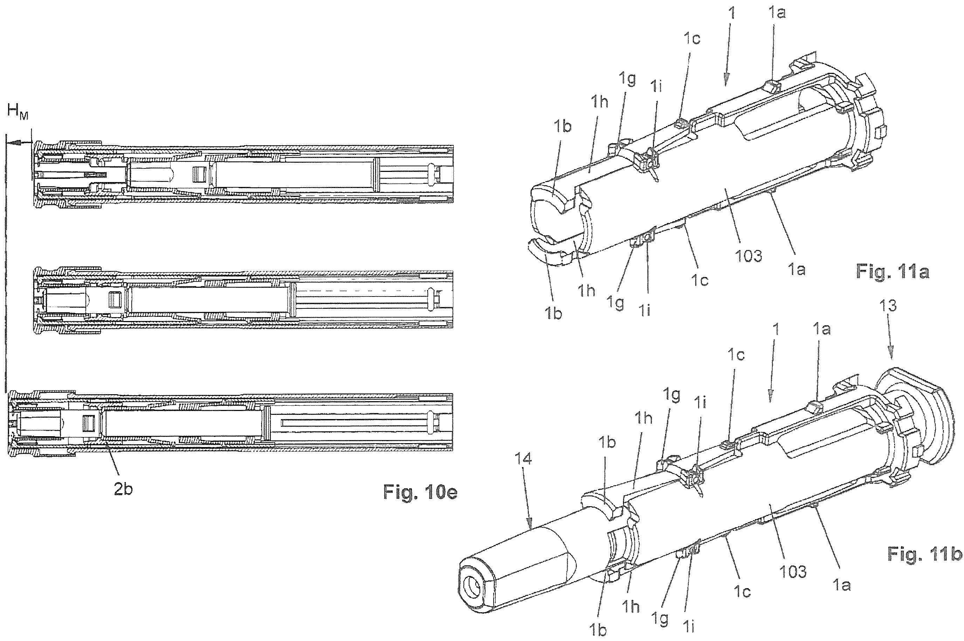

In der in den Figuren 10a-10d gezeigten Ausführung weist der Spritzenhalter 1 einen ersten Schalenkörper 101 und einen zweiten Schalenkörper 102 auf, die jeweils als Halbschale gebildet sind und insbesondere identisch ausgestaltet sind, damit Werkzeugkosten gespart werden können.

Jeder der ersten und zweiten Schalenkörper 101,102 weist einen Nocken 1c und eine Abragung 1a in der beschriebenen Weise auf. Ferner weist jeder der ersten und zweiten Schalenkörper 101, 102 an seinem distalen Ende ein Eingriffsglied 1b auf.

Jeder der Schalenkörper 101, 102 weist einen Scharnierzapfen 1e und eine Scharnierzapfenaufnahme 1f auf (Figur 10a), wobei der Scharnierzapfen 1e des einen Schalenkörpers 101, 102 in die Scharnierzapfenaufnahme 1f des anderen Schalenkörpers 102, 101 eingefügt wird (Figur 10b), so dass die erste und zweite Halbschale 101,102 relativ zueinander um die Schwenkachse des Schwenkgelenks 1e, 1f, welches aus den Scharnierzapfen 1e und den Schamierzapfenaufnahmen 1f gebildet wird, schwenkbar sind, nämlich zwischen einer Einfügeposition (Figur 10c) und einer Schliessposition (Figur 10d). Über das proximale Ende des Spritzenkörpers 1 wird die Spritze 13 zusammen mit der Nadelschutzkappe 14 eingefügt, wobei die Nadelschutzkappe 14 an dem Eingriffsglied 1b vorbeibewegt wird, wobei der erste Schalenkörper 101 und der zweite Schalenkörper 102 zueinander verschwenkt werden, wenn die Lücke zwischen der Nadelschutzkappe 14 und dem sich verjüngenden Bereich des Spritzenkörpers in Bezug auf die Längsachse L auf der gleichen Position wie die Eingriffsglieder 1b sind. Hierdurch greifen die Eingriffsglieder 1b in die genannte Lücke. Wie beschrieben können die Eingriffsglieder 1b von dem Halteabschnitt 2b des Gehäuses 2 in Eingriff mit dem sich verjüngenden Abschnitt des Spritzenkörpers gehalten werden. Alternativ oder zusätzlich können der erste Schalenkörper 101 und der zweite Schalenkörper 102 in der Schliessposition (Figur 10d), in der die Eingriffsglieder 1b in die Lücke eingreifen, miteinander verrasten.

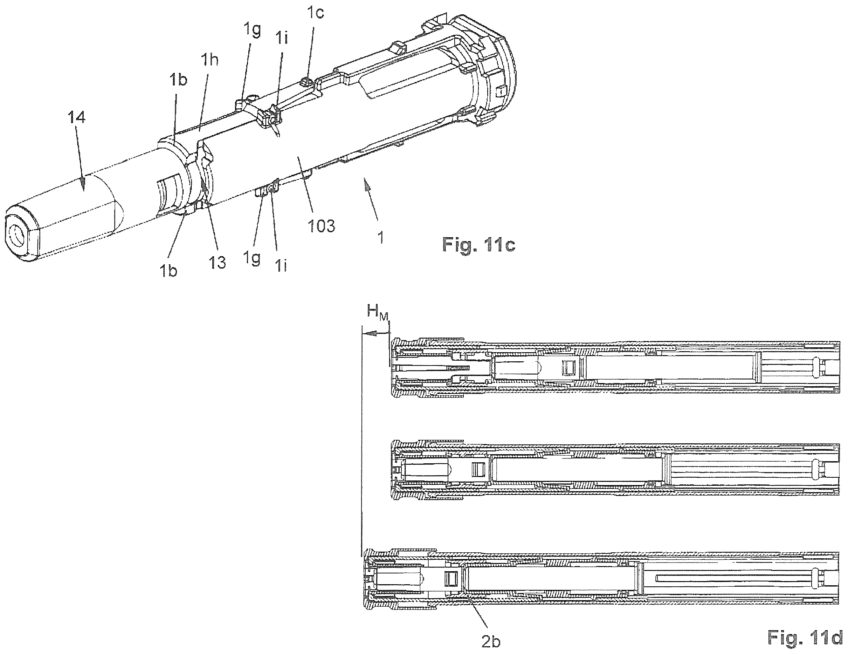

In den Figuren 11a bis 11c wird eine Ausgestaltungsform des Spritzenhalters 1 gezeigt, die einen ersten Hülsenkörper 103 und zwei Schwenkarme 1h aufweist. Die Abragung 1a ist an dem Hülsenkörper 103 gebildet. Der Hülsenkörper 103 bildet für jeden der Schwenkarme 1h zwei Schamierzapfenaufnahmen 1g, in denen jeweils ein Scharnierzapfen 1i des Schwenkhebels 1h angeordnet ist. Jeder der Schwenkhebel 1h bildet zwei Scharnierzapfen 1i, die mit der Scharnierzapfenaufnahme verrastet sind. Der Schwenkzapfen 1i ist relativ zu der Schwenkzapfenaufnahme 1g drehbar und kann an der Schamierzapfenaufnahme 1g gleiten. Der Schwenkhebel 1h weist einen Hebelabschnitt auf, der in die distale Richtung weist, wobei am distalen Ende dieses Hebelabschnitts das vom Schwenkhebel 1h gebildete Eingriffsglied 1b für den Eingriff in die Lücke zwischen der Nadelschutzkappe 14 und dem sich verjüngenden Abschnitt des Spritzenkörpers gebildet ist.

Der in dem Beispiel gezeigte Schwenkhebel 1h ist zweiarmig, wobei der Hebelabschnitt, der von dem Schwenkgelenk 1g, 1i in die entgegengesetzte Richtung wie der Arm, der das Eingriffsglied 1b bildet, abragt, den Nocken 1c bildet.

Die Spritze 13 wird mit der Nadelschutzkappe 14 voran über das proximale Ende des Hülsenkörpers 103 in den Hülsenkörper 103 eingeführt, wobei die Nadelschutzkappe 14 an den Eingriffsgliedern 1b vorbei bewegt wird, bis sich die Lücke zwischen dem sich verjüngenden Bereich des Spritzenkörpers und der Nadelschutzkappe 14 in Bezug auf die Längsachse auf der gleichen Position wie die Eingriffsglieder 1b befinden. Durch Verschwenken des Schwenkhebels 1h werden die Eingriffsglieder 1b in die Lücke bzw. zu der Längsachse hin geschwenkt. Die in Figur 11c gezeigte Einheit wird dann in dem Gehäuse 2 des Autoinjektors so angeordnet, dass der Halteabschnitt 2b den Schwenkhebel 1h so fixiert, dass die Eingriffsglieder 1b in dem Eingriff mit dem sich verjüngenden Abschnitt des Spritzenkörpers gehalten werden. Der Arm, an dem der Nocken 1c gebildet ist, ist in Bezug auf den Arm, an dem das Eingriffsglied 1b gebildet ist, elastisch verformbar, wodurch der Nocken 1c die in Bezug auf die Nadelschutzhülse 3 vorgesehene Aufgabe erfüllen kann. Insbesondere dient der Nocken 1a als Anschlag für die Nadelschutzhülse 3, wobei die Nadelschutzhülse 3 an dem Nocken 1a anliegt, wenn die Nadelschutzhülse in ihrer Ausgangsposition oder/und in ihrer Nadelschutzposition ist.

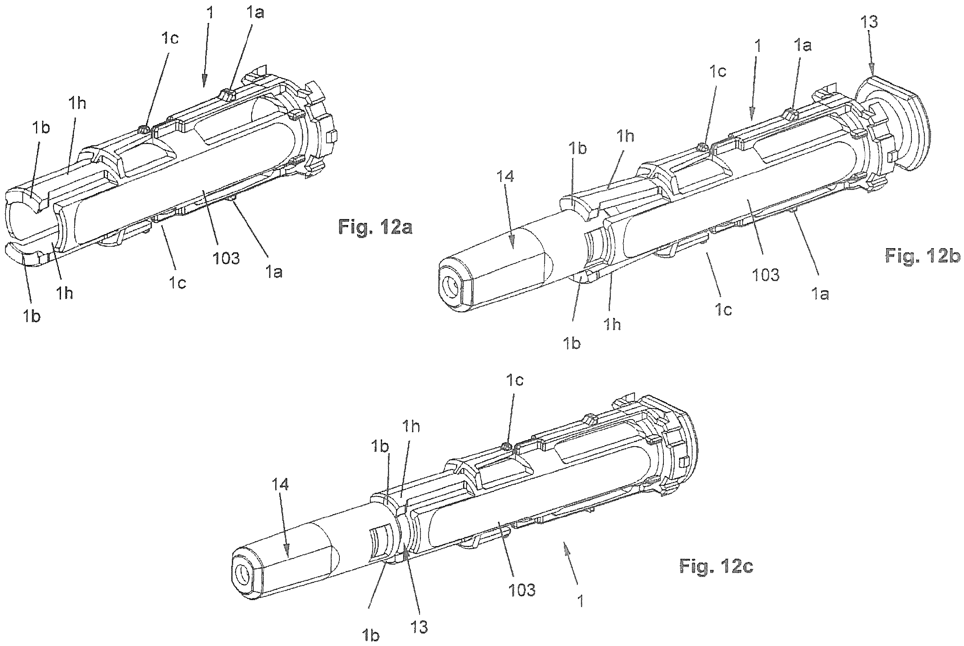

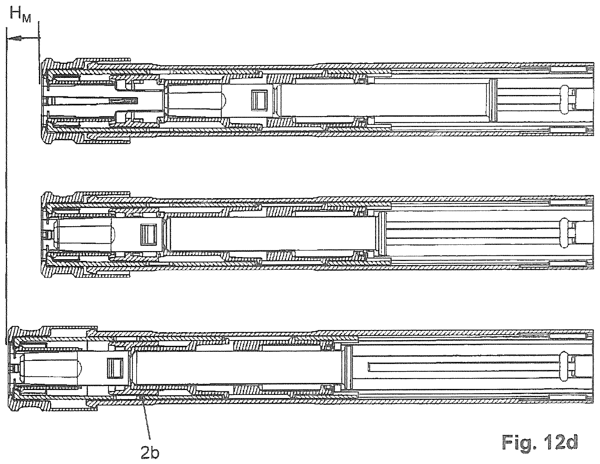

In der in den Figuren 12a-12d gezeigten fünften Ausgestaltungsform weist das Spritzenmodul, insbesondere der Spritzenhalter 1 einen Hülsenkörper 103 auf. Der Hülsenkörper 103 weist insbesondere zwei Nocken 1c und insbesondere zwei Abragungen 1a in der hierin gezeigten Weise auf.

In dieser Variante kann das mindestens eine Eingriffsglied als Schulter 1b fedemd, insbesondere an einem elastischen Arm 1h an dem Spritzenhalter gebildet sein, wobei die Spritze 13 über das proximale Ende mit der Nadel voraus in den Spritzenhalter, der vorzugsweise hülsenförmig ist, eingeschoben wird, wobei die Nadelschutzkappe 14 das mindestens eine Eingriffsglied 1b quer zur Längsachse nach aussen, d. h. von der Längsachse weg auslenkt, wobei, wenn die Nadelschutzkappe 14 vollständig an dem mindestens einen Eingriffsglied 1b vorbei bewegt wurde, das mindestens eine Eingriffsglied 1b in die Lücke zwischen dem sich verjüngenden Bereich der Spritze 13 und dem proximalen Ende der Nadelschutzkappe 14 einschnappt. Die in Figur 12c gezeigte Einheit wird dann in dem Gehäuse 2 des Autoinjektors so angeordnet, dass der Halteabschnitt 2b den Arm 1 h so fixiert, dass die Eingriffsglieder 1b in dem Eingriff mit dem sich verjüngenden Abschnitt des Spritzenkörpers 13 kraft- oder formschlüssig gehalten werden und aus diesem Eingriff nicht mehr herausfedern.

In den Figuren 8e, 9d , 10e, 11d, 12d sind Längsschnitte der fünf Ausgestaltungsformen im Auslieferungszustand und für die Ausgestaltungsformen zwei bis fünf in je nach einem Montageschritt der Spritzenmontage in den Autoinjektor in je einer Position mit teilweise und vollständig eingesetzter Spritze gezeigt. Bei vollständig eingesetzter Spritze greift ferner der zumindest eine Schnapper 4b welcher die Abziehkappe 4 aufweist in die Lücke zwischen dem Spritzenkörper 13, insbesondere dessen sich verjüngenden Bereich, und dem proximalen Ende des rigid needle shield 14 (Figur 2a, 2b).

Das zumindest eine Eingriffsglied 1b wird zusammen mit dem Spritzenhalter 1 durch einem Montagehub H_{M} welcher insbesondere als letzter Montageschritt erfolgt axial in den Bereich des Halteabschnittes 2b geschoben so dass eine kraft- oder formschlüssige Verbindung entsteht mit der verhindert wird, dass sich das mindestens eine Eingriffsglied 1b aus dem Eingriff mit dem verjüngenden Abschnitt des Spritzenkörpers 13 quer zur Längsachse, insbesondere von der Längsachse L weg oder nach aussen, bewegt. Weiter wird durch diesen Montagehub die Abziehkappe 4 in ihre distale Position bewegt, welche sie im Auslieferungszustand des Autoinjektors einnimmt, wobei die Abziehkappe 4 mittels zumindest einem Schnapphaken 4a welcher sich auf dem Spritzenhalter 1 abstützt durch den Spritzenhalter 1 bewegt wird.

Eine erfindungsgemässe Ausführungsform einer Injektionsvorrichtung ist in Figur 13 abgebildet. Der abgebildete Autoinjektor 200 entspricht weitgehend dem oben beschriebenen Autoinjektor, weshalb auf die obige Beschreibung verwiesen wird. Autoinjektor 200 weist ein Gehäuse 202 auf, welches sich vom Gehäuse 2 unterscheidet. Gehäuse 202 weist mehrere Befestigungspunkte 202f auf, welche die Form von Vertiefungen aufweisen. Die Befestigungspunkte 202f sind beidseitig auf dem Gehäuse vorhanden, wobei in Figur 13 nur eine Seite sichtbar ist. Alternativ könnten die Befestigungspunkte 202f anstatt als punktförmige Vertiefungen auch als axial orientierte Kanäle ausgebildet sein, ohne dass die Erfindungsidee dadurch beeinflusst würde.

Die erfindungsgemässe Injektionsvorrichtung dieser Ausführungsform umfasst zwei gehäuseartige Schalen 230, welche identisch sind (in Figur 13 ist nur eine davon abgebildet). Die Schalen 230 weisen jeweils ein offenes distales Ende 230d auf sowie ein in dieser Ausführungsform geschlossen ausgeformtes proximales Ende 230e. Die Schalen 230 umfassen mehrere Haltevorrichtungen 230a, welche als Schnapparme ausgebildet sind, an deren freiem Ende jeweils ein Zahn ausgebildet. Weiter umfassen die Schalen 230 mehrere Blockiervorrichtungen 230b, welche als Balken oder Rippen ausgebildet sind. Beide, die Schnapparme 230a und die Blockiervorrichtungen 230b, werden je nach Position und der damit verbundenen Dimension der Schalen 230 durch Stege 230f respektive 230g an den Schalen abgestützt, wobei die Stege 230f respektive 230g wie in der Figur 13 gezeigt variable Dimensionen haben können. Die Schalen können zur Optimierung der mechanischen Eigenschaften weitere Stege 230h umfassen.

Die Schalen 230 sind so dimensioniert, dass sie auf das Gehäuse 202 aufgeschnappt werden können. Dabei verschnappen die Zähne der Schnapparme 230a mit den Vertiefungen der Befestigungspunkte 202f. Die Blockiervorrichtungen 230b sind so auf den Schalen 230 angeordnet, dass sie nach dem Verschnappen der Schalen 230 mit dem Gehäuse 202 axial jeweils auf gleicher Höhe wie eine Haltevorrichtung zu liegen kommen, dabei radial weiter aussen liegen als die Schnapparme 230a und somit eine radiale Auswärtsbewegung der Schnapparme 230a blockieren können. Diese Situation ist in Figur 14a dargestellt. Figur 14a zeigt die erfindungsgemässe Injektionvorrichtung 200 in zusammengebautem Zustand, als nach dem Anbringen der beiden gehäuseartigen Schalen 230 am Gehäuse 202. Um der Klarheit willen sind die beiden identischen Schalen 230 in unterschiedlichen Grautönen dargestellt. Die Blockiervorrichtungen 230b blockieren im in Figur 14 a abgebildeten Zustand eine Bewegung der Schnapparme 230a in die Pfeilrichtung (siehe Figur 14a), also radial nach aussen und bewirken so eine unlösbare Verbindung zwischen den Schalen 230 und dem Gehäuse 202.

Eine vorteilhafte Eigenschaft dieser Ausführungsform ist in Figur 14b dargestellt, wobei die Injektionsvorrichtung auf eine Auflage, zum Beispiel einem Tisch 1000 liegt. Der in Figur 14b dargestellte Querschnitt zeigt den ovalen Querschnitt der Schalen 230 und die Formung der Fensteröffnung 230c. Die gezeigte Ausgestaltung hat hier den Vorteil, dass die Injektionsvorrichtung 200 aufgrund der Formgebung nicht wegrollen kann, wie es bei einer kreiszylindrischen Ausgestaltung des Querschnitts passieren könnte. Der Elemente zum Anbringen und unlösbar Befestigen der Schalen 230 am Gehäuse 202 sind in Figur 15 noch einmal detailliert abgebildet.

Eine weitere erfindungsgemässe Ausführungsform ist in Figur 16 gezeigt. Die Injektionsvorrichtung 300 dieser Ausführungsform umfasst unter anderem ein Gehäuse 302, auf welchem die Befestigungspunkte 302f angeordnet sind, welche kleine, zahnartige Strukturen aufweisen, die in den leicht angesenkten Befestigungspunkten hervorstehen. Weiter umfasst die Injektionsvorrichtung dieser Ausführungsform zwei gehäuseartige Schalen 330. Die Schalen weisen beispiel- und vorteilhaft auf ihrer Aussenfläche eine Oberflächenstrukturierung 330i auf, welche der bedienenden Person einen sicheren Griff ermöglichen soll. Die Schalen 330 umfassen mehrere Haltevorrichtungen 330a, welche als Arme ausgebildet sind. An ihrem freien Ende ist jeweils eine Öffnung angeordnet, Analog zur vorherig beschriebenen Ausführungsform umfassen die Schalen 330 jeweils mehrere Blockiervorrichtungen 330b. Die Arme 330a können beim Anbringen der Schalen 330 an das Gehäuse 302 mit den Zähnen an den Befestigungspunkten 302f verschnappen. Analog zur vorherigen Ausführungsform blockieren die Blockiervorrichtungen 330b ein Lösen der Arme 330a aus der Verschnappung mit den Befestigungspunkten. Die mechanischen Eigenschaften der Arme 330a können dabei über Dimensionierung der Rippen 330f variiert werden.

Die Figuren 17a-d stellen vier mögliche Ausgestaltungen der gehäuseartigen Schalen dar, welche auf den jeweiligen Gehäusen angebracht sind. In Figur 17a ist eine Art Minimalvariante der Schale dargestellt. Schale 430 weist eine in etwa kreiszylindrische Form, wobei Schale 430 eine Halbschale ist. Die Oberfläche der Schale 430 weist im proximalen Bereich eine Oberflächenstrukturierung auf. In Figur 17b ist eine ergonomisch geformte Variante einer gehäuseartigen Schale abgebildet. Die Schale 330 weist eine Setzholz- oder Pikierstab-artige Form auf, welche ebenfalls im proximalen Bereich 330i strukturiert ist. Die Schale 230 sieht äusserlich ein anders aus als Schale 330; sie besteht aus zwei Materialkomponenten, welche im Zweikomponenten-Spritzgussverfahren hergestellt wurde. Der proximale Teil besteht bei Schale 230 aus einem weicheren Material als der distale Teil. Das weichere Material soll die Griffigkeit für die benutzende Person verbessern. Alternative Varianten für zweikomponentige Schalen könnten zum Beispiel auch verschiedene Farben der beiden Komponenten sein. Figur 17d zeigt eine gehäuseartige Schale 530 mit zielgruppenorientierter Designanpassung in Form von dekorativen Sternen. Die als grosser Stern 535 ausgebildete Struktur am proximalen Ende der Injektionsvorrichtung kann auch andere Formen haben. Zum Beispiel kann es sich um tastbare Symbole handeln, welche sehbehinderten benutzenden Personen die Identifikation der Injektionsvorrichtung erleichtern oder gar ermöglichen.

In den Figuren 18a-b sind weitere vorteilhafte Ausgestaltungmöglichkeiten der gehäuseartigen Schalen dargestellt. So hat im Beispiel der Figur 18a die Fensteröffnung 230c etwa die gleiche Länge wie die darunterliegende Öffnung 202h des Gehäuses. Die Öffnung 202h ermöglicht den Blick auf den Produktbehälter 13 und dessen Füllstand. Im Beispiel der Figur 18a erlaubt die Fensteröffnung 230c in etwa denselben Sichtbereich wie die Öffnung 202h. Im Beispiel der Figur 18b ist die Fensteröffnung 630c deutlich kürzer, so dass nur ein Teilbereich der Öffnung 202h dem Auge der benutzenden Person zugänglich gemacht wird. Diese Variante der gehäuseartigen Schale kann zum Beispiel dann vorteilhaft sein, wenn der Produktbehälter 13 der Injektionsvorrichtung 2 absichtlich nur mit Teilmengen der möglichen maximalen Füllmenge befüllt ist. So kann das gleiche Gehäuse 202 für verschiedene Füllmengen verwendet werden ohne der benutzenden Person den Eindruck zu geben, dass der Produktbehälter ungenügend gefüllt sei.

In Figur 19 ist noch einmal das Gehäuse 202 abgebildet. Dabei sind in Figur 19 die Gehäuseöffnungen 202a und 202g sichtbar. Bei diesen Öffnungen kann es sich zum Beispiel um Montagehilfen oder Fixieranordnungen für Mechanikteile, Teile der Produktbehälterhalterung, Tasteröffnungen, Sensoriköffnungen, Lichtleiterzugänge, transparente Stellen oder Zugänge für elektrische Kontakte handeln. Diese Öffnungen muss die benutzende Person nicht sehen um die Injektionsvorrichtung benutzen können. Im Gegenteil, solche Öffnungen können die benutzende Person verwirren oder gar zu Fehlmanipulationen verleiten, weshalb die gehäuseartigen Schalen diese Öffnungen vorteilhaft abdecken.

In den Figuren 20a-22 ist eine weitere erfindungsgemässe Ausführungsform der Injektionsvorrichtung abgebildet. Diese Ausführungsform umfasst die Injektionsvorrichtung 700, beispielhaft als Autoinjektor analog dem Autoinjektor 200 dargestellt. Die Injektionsvorrichtung 700 umfasst ein Gehäuse 702. Auf der äusseren Oberfläche des Gehäuses 702 sind mehrere Befestigungspunkte 702f (als Vertiefungen ausgebildet) angeordnet.

Weiter umfasst die Injektionsvorrichtung 700 vier identische gehäuseartige Schalen 730, welche unlösbar am Gehäuse 702 anbringbar sind, Dazu umfassen die Schalen 730 jeweils mehrere Haltevorrichtungen 730a sowie jeweils mehrere Blockiervorrichtungen 730b. Die Schalen 730 sind in dieser Ausführungsform als Zylindersegmentschalen ausgebildet, welche einen Winkel von etwa 90° überstreichen, also in etwa einen Viertel eines Kreises im Querschnitt. In der vorliegenden Ausführungsform haben die Schalen etwa die halbe Länge der Injektionsvorrichtung 700 und werden in der proximalen Hälfte der Injektionsvorrichtung angebracht. Alle vier Schalen zusammen umschliessen den proximalen Teil der Injektionsvorrichtung ziemlich vollständig. Stellt man sich einen kompletten Zylinder aus vier der Schalen 730 vor, so sind die Haltevorrichtungen 730a sowie die Blockiervorrichtungen 730b ins Zylinderinnere in radiale Richtung ausgerichtet und als kurze Arme ausgebildet.

Die einzelnen Schalen 730 können auf das Gehäuse 702 aufgeschnappt werden, wenn die Haltevorrichtungen 730a und die Blockiervorrichtungen 730b vorgängig korrekt auf die Befestigungspunkte 702f ausgerichtet wurden. Die Anordnung von Haltevorrichtungen 730a und Blockiervorrichtungen 730b an den Schalen 730 sind dabei in Figur 22 dargestellt. Sie sind jeweils entlang eines Längsrandes einer Schale angeordnet, in diesem Beispiel die Haltevorrichtungen 730a auf einer Seite und Blockiervorrichtungen 730b auf der anderen Seite.

Um eine Schale 730 auf das Gehäuse 702 aufschnappen zu können, müssen die Aussenkanten leicht nach aussen gebogen werden, damit die Arme von Haltevorrichtungen 730a und Blockiervorrichtungen 730b über die Kanten 730l der Vertiefungen der Befestigungspunkte 702f gleiten können. Nachdem eine Schale 730 auf das Gehäuse 702 aufgeschnappt wurde, kann die nächste Schale 730 auf das Gehäuse 702 aufgeschnappt werden. Dabei werden die Blockiervorrichtungen 730b an Befestigungspunkten 702f eingebracht, in welche schon Haltevorrichtungen 730a der vorherigen Schale 730 eingebracht worden sind. Um das Einbringen der Blockiervorrichtungen 730b zu vereinfachen, ist am freien Ende der Arme eine Fase 730k angebracht. Sind die Blockiervorrichtungen 730b eingebracht, so ist es nicht mehr möglich die Haltvorrichtungen 730a zu lösen, Sind sodann alle vier Schalen 730 auf der Oberfläche des Gehäuses 702 angebracht, i. e. aufgeschnappt, so sind sie in Konsequenz nicht mehr zerstörungsfrei vom Gehäuse entfernbar.

Die Figuren 20b und 20c zeigen die erfindungsgemässe Injektionsvorrichtung 700 in einem Zustand, in welchem alle vier Schalen 730 aufgeschnappt sind. Der in Figur 20c markierte Querschnitt ist in Figur 21a dargestellt. Figur 21b zeigt vergrössert einen Befestigungspunkt 702f, in welchem eine Haltevorrichtung 730a sowie eine Blockiervorrichtung 730b angebracht sind.

Die vier gehäuseartigen Schalen 730 sind im erklärten Beispiel identisch. In weiteren Ausgestaltungen der Ausführungsform können sich die Schalen unterscheiden, insbesondere in Form und Material. Dies ist so lange möglich, als die Haltevorrichtungen 730a und Blockiervorrichtungen 730b nach wie vor so angeordnet sind, dass sie geometrisch und formlich zu den Befestigungspunkten 702f des Gehäuses 702 passen.

In den Figuren 23a bis 24b ist eine weitere vorteilhafte Ausgestaltung einer erfindungsgemässen Injektionsvorrichtung. Die Injektionsvorrichtung 800 umfasst zwei gehäuseartige Schalen 830 und 830', welche am Gehäuse 802 angebracht werden können und zusammen das Gehäuse 802 in etwa überdecken. Die gehäuseartigen Schalen 830 und 830' werden analog der Schalen 330 am Gehäuse 802 angebracht. Es sei an dieser Stelle erwähnt, dass die Schalen 330 auch auf das Gehäuse 802 passen würden. Die Schale 830' weist die gleiche Form auf wie die Schale 830 verfügt jedoch über ein zusätzliches Elektronikmodul 840. Das Elektronikmodul 840 ist auf der Innenseite der Schale 830' angebracht, zum Beispiel angeklebt oder verschnappt. Die Schale 830' weist Öffnungen 830m, 830n und 830o auf, durch welche Elemente des Elektronikmoduls von aussen sichtbar oder zugänglich sind.

Die Figuren 24a und 24b zeigen die Details des in Schale 830' integrierten Elektronikmoduls 840. Figur 24a eine erste Seite des Elektronikmoduls. Als zentrale Steuereinheit ist der Mikrokontroller 840f vorgesehen. Dieser steuert die zusätzlichen Bauteile an. So umfasst das Elektronikmodul 840 über ein Sensormodul 840i, welches beispielhaft Temperatur-, Feuchtigkeits-, Licht-, Drucksensoren und/oder ein Mikrophon umfassen kann. Ebenfalls auf dieser Seite des Elektronikmoduls 840 sind weiter ein Lautsprecher oder Summer 840e als akustischer Emitter sowie das Kommunikationsmodul 840j zur drahtlosen Kommunikation mit externen Geräten vorgesehen. Die Energieversorgung ist ebenfalls direkt auf dem Elektronikmodul 840 vorgesehen, dies beispielhaft in Form der Batterien 840g. Die rückwärtige Seite des Elektronikmoduls ist in Figur 24b gezeigt. Dort angeordnet sind ein Taster oder Knopf 840d, zwei LEDs 840b und 840c sowie das Display 840a. Die Leuchtdiode 840b ist dabei als grüne LED ausgeführt und die Leuchtdiode 840c also rote LED ausgeführt, wobei die Farbgebung auch anders sein könnte. Das Display 840a ist als einfaches 7-Segment Display ausgeführt mit zwei Stellen. Es könnte jedoch auch höher auflösendes LCD oder OLED Display verwirklicht sein. Taster 840d, die LEDs 840b und 840c sowie das Display 840a, sind durch die entsprechenden Öffnungen 830o, 830n und 830m in der Schale 830' von aussen zugänglich respektive sichtbar.

Mit dem Elektronikmodul 840 verbunden ist der Sensor 840h. Wie in den Figuren 24a und 24b zu sehen umfasst der Sensor 840h einen Stift 840k. Dieser Stift 840k ist am Sensor 840h beweglich gelagert. Werden die Schalen 830 und 830' am Gehäuse 802 angebracht, so wird der Stift 840k durch eine Rippe an der Schale 830 (nicht gezeigt) in den Sensor 840h hinein bewegt und schliesst einen im Sensor 840h vorhandenen elektrischen Kontakt. Dieser Kontaktschluss wird vom Mikrokontroller 840f ausgewertet. Im vorliegenden Beispiel bedeutet der Kontaktschluss, dass die Injektionsvorrichtung inklusive einem Produktbehälter fertig montiert ist, da die gehäuseartigen Schalen 830 und 830' die letzten Teile sind, welche an der Injektionsvorrichtung. Als Folge des Kontaktschlusses aktiviert der Mikrokontroller einen internen Lebensdauertimer sowie die vorhandenen Sensoren 840i, insbesondere einen vorhandenen Temperatursensor. Der Lebensdauertimer signalisiert nach dessen Ablauf, wenn die Injektionsvorrichtung nicht mehr verwendet werden sollte, da das Ablaufdatum des im Produktbehälter vorhandenen Medikamentes abgelaufen ist. Der Timer wird während der Montage deshalb spezifisch für das verwendete Medikament und dessen Haltbarkeit programmiert. Die Signalisierung erfolgt einerseits über die rote LED 840c optisch, welche insbesondere nach Ablauf blinken kann, sowie über den Lautsprecher 840e, welcher akustische Signale emittieren kann. Der erwähnte Temperatursensor im Sensor 840i misst die Temperatur der Injektionsvorrichtung, wobei das entsprechende Signal vom Mikrokontroller 840f ausgewertet und in einem internen Speicher (nicht explizit gezeigt) ablegt. Überschreitet oder unterschreitet die Temperatur der Injektionsvorrichtung über ein gewisse Dauer einen vorgegebenen Wert und muss dann davon ausgegangen werden, dass das Medikament durch den Impakt in Mitleidenschaft gezogen wurde, so aktiviert der Mikrokontroller, LEDs, Lautsprecher und/oder das Display, um nach aussen zu signalisieren, dass die Injektionsvorrichtung nicht mehr eingesetzt werden sollte. Dabei unterscheiden sich die emittierten Signale von denjenigen, welche nach Ablauf der Lebensdauer emittiert werden.

Das Sensormodul 840i umfasst im vorliegenden Beispiel weiter ein Mikrophon, mit welchem der Mikrokontroller 840f detektieren kann, wenn die Injektionsvorrichtung 800 für eine Injektion benutzt wird. Das Auslösen der Injektionsvorrichtung 800 und die damit verbundenen Bewegungen im Ausschüttmechanismus emittieren ein spezifisches akustisches Muster, welches vom Mikrokontroller 840f erkannt wird. Der Mikrokontroller 840f ist nun so programmiert, dass er der benutzenden Person die Injektion signalisiert und dieser anschliessend auch signalisiert, wenn der Injektionsvorgang beendet ist (und zum Beispiel die Injektionsnadel aus dem Gewebe entfernt werden kann. Im vorliegenden Beispiel beginnt die grüne LED 840b zu blinken, sobald der Mikrokontroller das Auslösen der Injektionsvorrichtung erkannt, die optische Anzeige kann dabei von akustischen Signalen aus dem Lautsprecher 840e unterstützt (auch durch Sprachausgabe wie: "Die Injektion läuft, bitte warten"). Gleichzeitig mit der LED wird das Display 840a aktiviert und die vorgesehene Dauer der Injektion wird in Sekunden zurückgezählt, um der benutzenden Person einen Anhaltspunkt darüber zu geben, wie lange der Injektionsvorgang noch dauern wird. Kommt der Zähler bei null an, so ist der injektionsvorgang abgeschlossen. Die grüne LED 840b blinkt nun nicht mehr, sondern leuchtet kontinuierlich als Zeichen für den Abschluss. Ebenso kann weiter eine akustische Rückmeldung über den Lautsprecher 840e erfolgen, wie zum Beispiel eine Sprachausgabe "Injektionsvorgang abgeschlossen". Der Mikrokontroller registriert und speichert Zeit und Datum des injektionsvorgangs.

Zusätzlich zu den bereits ausführlicher beschriebenen Teilen des Elektronikmoduls 840 kann das Elektronikmodul fakultativ ein Kommunikationsmodul 840j umfassen, welches mittels Bluetooth, WLAN oder GSM Daten aus der Injektionsvorrichtung 800 heraus versenden oder Daten resp. Befehle von extern empfangen kann. Im vorliegenden Beispiel kann der Mikrokontroller 840f über das Kommunikationsmodul 840j via Bluetooth Zeit und Datum des Injektionsvorganges sowie eine eindeutige Kennung der Injektionsvorrichtung 800 an ein Smartphone übertragen. Eine zugehörige App des Smartphones kann sodann Protokoll über eine Therapie führen. Die eindeutige Kennung der Injektionsvorrichtung erlaubt einen Rückschluss auf das Medikament, das Medikamentenlot, den genauen Typ der Injektionsvorrichtung sowie dem Produktionslot der Injektionsvorrichtung. Die von der App gesammelten Daten können sodann an den behandelnden Arzt, den Versicherer oder an einen Server im Internet übertragen werden.

Der Taster 840d hat zwei Funktionen, einerseits kann durch kurzes Drücken des Tasters 840d eine Lebensdauerüberprüfung resp. Funktionskontrolle vorgenommen werden. Wird der Taster 840d kurz gedrückt, prüft der Mikrokontroller 840f einerseits ob der Timer läuft und anderseits, ob das Sensormodul 840i plausible Messwerte liefert, in dem der Mikrokontroller 840f die aktuellen Messwerten gegen eine hinterlegte Bibliothek von Messwerten abgleicht. Ist beides der Fall, gibt der Mikrokontroller 840f über die grüne LED 840b und den Lautsprecher 840a eine entsprechende Bestätigung aus. Ergibt eine der Prüfungen ein Problem so, gibt der Mikrokontroller eine Rückmeldung über die rote Led 840c sowie den Lautsprecher aus. Damit kann eine benutzende Person jederzeit überprüfen, ob eine Injektionsvorrichtung noch verwendbar ist oder nicht.

Weitere vorteilhafte Ausführungsformen sind dem Fachmann direkt ohne erfinderische Tätigkeit zugänglich, in dem er die Zahl der gehäuseartigen Schalen respektive die Ausgestaltung der Befestigungspunkte, der Haltevorrichtungen sowie der Blockiervorrichtungen im Rahmen der Lehre und seines Fachwissens variiert.

### 7 Bezeichner

### Bezugszeichenliste:

- 1: Spritzenhalter
- 1a: Abragung/ Halteglied
- 1b: Schulter/ Eingriffsglied
- 1c: Nocken
- 1e: Scharnierzapfen
- 1f: Scharnierzapfenaufnahme
- 1g: Scharnierzapfenaufnahme
- 1i: Schamierzapfen
- 1h: Schwenkhebel/Arm
- 1k: Translationsgegenanschlag
- 1m: Translationsanschlag
- 2: Gehäuse
- 2a: Ausnehmung
- 2b: Ringabschnitt/Halteabschnitt
- 2c: Betätigungsanschlag
- 2e: Halteschulter
- 3: Nadelschutzhülse
- 3a: proximales Ende
- 3b: Schnappgeometrie
- 4: Abziehkappe
- 4a: Schnapphaken
- 4b: Schnapper
- 5: Mechanikhalter
- 5a: Startsignalanschlag
- 5b: Nut
- 5c: Haltefederabschnitt
- 5d: Vertiefung
- 6: Halteelement
- 6a: erstes Eingriffselement
- 6b: zweites Eingriffselement
- 6c: Arm
- 6d: Führungsstift
- 7: Vortriebsglied
- 7a: erste Ausnehmung
- 7b: zweite Ausnehmung
- 8: Sperrhülse
- 8a: Verriegelungsglied
- 8b: erste Ausnehmung
- 8c: zweite Ausnehmung
- 9: erste Feder/Ausschüttfeder
- 10: zweite Feder/Nadelschutzfeder
- 11: Signalglied
- 11a: erstes Eingriffsglied
- 11b: zweites Eingriffsglied
- 11c: Abragung
- 11: d Arm
- 12: Verschlusskappe
- 12a: Rastglied
- 12b: Signalanschlag
- 13: Produktbehälter/Spritze
- 13a: Nadel
- 13b: Kolben
- 14: rigid needle shield/ Nadelschutzkappe
- 15: Schalthülse
- 15a: Ausnehmung
- 101: erster Schalenkörper/Halbschale
- 102: zweiter Schalenkörper/Halbschale
- 103: erster Schalenkörper/Hülsenkörper
- 104: zweiter Schalenkörper/ Hülsenkörper
- 200: Injektionsvorrichtung
- 202: Gehäuse
- 202a: Öffnung
- 202f: Befestigungspunkt/Schnappvertiefung
- 202g: (Gehäuse-)Öffnung
- 202h: Fenster oder Öffnung
- 230: Schale
- 230a: Schnapparm mit Schnapper
- 230b: Blockiervorrichtung(en)
- 230c: Fensteröffnung
- 230d: distales Ende der Schale
- 230e: proximales Ende der Schale
- 230f: Stege
- 230g: Stege
- 230h: Stege
- 300: Injektionsvorrichtung
- 302: Gehäuse
- 302f: Befestigungspunkt mit Schnappzahn
- 330: Schale
- 330a: Arm
- 330b: Blockiervorrichtung(en)
- 330f: Rippen
- 330i: Oberflächenstrukturierung
- 430: Schale
- 530: Schale
- 535: Stern
- 630: Schale
- 630c: Fensteröffnung
- 700: Injektionsvorrichtung
- 702: Gehäuse
- 702f: Befestigungspunkt
- 730: Schale
- 730a: Haltevorrichtung
- 730b: Blockiervorrichtung(en)
- 730e: proximales Ende der Schale
- 730k: Fase
- 730l: Kante
- 800: Injektionsvorrichtung
- 802: Gehäuse
- 802f: Befestigungspunkt
- 830: Schale
- 830a: Haltevorrichtung
- 830b: Blockiervorrichtung
- 830m: Öffnung für Display
- 830n: Öffnungen für LEDs
- 830o: Öffnung für Taster
- 840: Elektronikmodul
- 840a: Display
- 840b: LED
- 840c: LED
- 840d: Knopf/Taster
- 840e: Lautsprecher/Summer
- 840f: Mikrokontroller
- 840g: Batterie(n)
- 840h: Sensor
- 840i: Sensor
- 840j: Kommunikationsmodul
- 840k: Stift
- 1000: Tisch (nicht vollständig abgebildet)
- H_{A}: Ausschütthub
- H_{B}: Betätigungshub
- H_{S}: Signalhub/erster Teilhub
- H_{K}: Startsignalhub
- H_{N}: Nadelschutzhub
- H_{M}: Montagehub
- L: Längsachse

## Patentansprüche

1. Eine Injektionsvorrichtung (200) zum Verabreichen einer fluiden Substanz mindestens umfassend ein Gehäuse (202), welches auf seiner Aussenseite eine Anordnung von mehreren Befestigungspunkten (202f) aufweist,
die Injektionsvorrichtung (200) weiter umfassend
mindestens zwei gehäuseartige Schalen (230), an welchen jeweils
mindestens eine Haltevorrichtung (230a) und
mindestens eine Blockiervorrichtung (230b) angeordnet sind,
wobei die Schalen (230) auf dem Gehäuse (202) anbringbar sind, **dadurch gekennzeichnet, dass** die Haltevorrichtung (230a) geometrisch so angeordnet ist, dass sie beim Anbringen der Schale (230) am Gehäuse (202) in einem Befestigungspunkt (202f) zu liegen kommt und damit zur Befestigung der Schale (230) am Gehäuse (202) beiträgt und wobei
die mindestens eine Haltevorrichtung (230a) und die Blockiervorrichtung (230b) so an den Schalen (230) angeordnet sind, dass
mindestens eine Haltevorrichtung (230a) einer ersten Schale (230) am Befestigungspunkt (202f) anbringbar ist,
mindestens eine Haltevorrichtung (230a) einer zweiten Schale (230) an einem weiteren Befestigungspunkt (202f) anbringbar ist,
die mindestens eine, an einem Befestigungspunkt (202f) angebrachte Haltevorrichtung (230a) der ersten Schale (230) durch eine Blockiervorrichtung (230b) der zweiten Schale (230) an einem Lösen vom Befestigungspunkt (202f) gehindert wird,
die mindestens eine, an einem weiteren Befestigungspunkt (202f) angebrachte Haltevorrichtung (230a) der zweiten Schale (230) durch eine Blockiervorrichtung (230b) einer weiteren Schale (230) an einem Lösen vom weiteren Befestigungspunkt (202f) gehindert wird oder, falls die Injektionsvorrichtung (200) nur zwei Schalen (230) umfasst, durch eine Blockiervorrichtung (230b) der ersten Schale (230), und
die mindestens eine, an einem weiteren Befestigungspunkt (202f) angebrachte Haltevorrichtung (230a) der weiteren Schale (230) durch eine Blockiervorrichtung (230b) einer nächsten Schale (230) an einem Lösen vom weiteren Befestigungspunkt (202f) gehindert wird und so weiter, bis eine Haltevorrichtung (230a) der letzten Schale an einem Befestigungspunkt (202f) des Gehäuses (202) angebracht wird, welche durch eine Blockiervorrichtung (230b) der ersten Schale (230) an einem Lösen vom Befestigungspunkt (202f) gehindert wird, wodurch
die Schalen (230) unlösbar mit dem Gehäuse (202) verbunden werden.

2. Eine Injektionsvorrichtung (200) nach Anspruch 1, die Injektionsvorrichtung (200) genau zwei Schalen (230) umfasst.

3. Eine Injektionsvorrichtung (200) nach Anspruch 1 oder 2, wobei die Schalen (230) geometrisch identisch sind.

4. Eine Injektionsvorrichtung (200) nach Anspruch 1 oder 2, wobei die Schalensegmente identisch sind.

5. Eine Injektionsvorrichtung (200) nach einem der vorhergehenden Ansprüche, **dadurch gekennzeichnet, dass** es sich bei den Haltevorrichtungen (230a) um Schnapparme mit einer zahnartigen Struktur am freien Ende handelt und bei den Befestigungspunkten (202f) um zur zahnartigen Struktur komplementär ausgebildeten Vertiefungen.

6. Eine Injektionsvorrichtung (200) nach einem der Ansprüche 1 bis 4, **dadurch gekennzeichnet, dass** es sich bei den Haltevorrichtungen (230a) um Arme handelt, welche an ihrem freien Ende eine Öffnung aufweisen, und die Befestigungspunkte (202f) zahnartige Strukturen umfassen, wobei die Haltevorrichtungen (230a) mit den Befestigungspunkten (202f) verschnappen können.

7. Eine Injektionsvorrichtung (200) nach Anspruch 1, die Injektionsvorrichtung (200) genau drei Schalen (230) umfasst.

8. Eine Injektionsvorrichtung (200) nach Anspruch 1, die Injektionsvorrichtung (200) genau vier Schalen (230) umfasst.

9. Eine Injektionsvorrichtung (200) nach Anspruch 1, wobei das Gehäuse (202) zumindest eine Öffnung (202h) umfasst, welche die Sicht auf einen Produktbehälter (13) freigibt, welcher die zu verabreichende Substanz enthält und zumindest eine gehäuseartige Schale (230), welche im Bereich der Gehäuseöffnung am Gehäuse (202) angebracht wird, ebenfalls eine Öffnung (230c) umfasst, **dadurch gekennzeichnet, dass** die Öffnung (230c) in der gehäuseartigen Schale (230) im Bereich der Gehäuseöffnung zu liegen kommt, wenn die Schale (230) am Gehäuse (202) angebracht ist, so dass der Produktbehälter (13) auch bei angebrachter Schale (230) zumindest teilweise sichtbar bleibt.

10. Eine Injektionsvorrichtung (200) nach dem vorhergehenden Anspruch, **dadurch gekennzeichnet, dass** die Öffnung (202c) in der gehäuseartigen Schale (230) kleiner ist, als die Öffnung (202h) im Gehäuse (202).

11. Eine Injektionsvorrichtung (200) nach Anspruch 1, **dadurch gekennzeichnet, dass** die Befestigungspunkte (202f) optisch oder elektrisch leitende Bereiche aufweisen.

12. Eine Injektionsvorrichtung (200) nach dem vorhergehenden Anspruch, **dadurch gekennzeichnet dass** mindestens eine Haltevorrichtung optisch oder elektrisch leitende Elemente aufweist, welche die optisch respektive elektrisch leitenden Bereiche der Befestigungspunkte (202f) kontaktieren können.

## Claims

1. An injection device (200) for administering a fluid substance including at least a housing (202) which on its outside has an arrangement of a plurality of fixing points (202f),
the injection device (200) further including
at least two housing-like shells (230) on which there are respectively are arranged
at least one holding device (230a), and
at least one blocking device (230b),
wherein the shells (230) can be mounted on the housing (202), **characterised in that** the holding device (230a) is geometrically so arranged that when the shell (230) is mounted to the housing (202) the holding device comes to lie in a fixing point (202f) and thus contributes to fixing the shell (230) to the housing (202), and wherein
the at least one holding device (230a) and the blocking device (230b) are so arranged on the shells (230) that
at least one holding device (230a) of a first shell (230) can be mounted to the fixing point (202f),
at least one holding device (230a) of a second shell (230) can be mounted at a further fixing point (202f),
the at least one holding device (230a) of the first shell (230), that is mounted to a fixing point (202f), is prevented by a blocking device (230b) of the second shell (230) from release from the fixing point (202f),
the at least one holding device (230a) of the second shell (230), that is mounted at a further fixing point (202f), is prevented by a blocking device (230b) of a further shell (230) from release from the further fixing point (202f) or if the injection device (200) includes only two shells (230) by a blocking device (230b) of the first shell (230), and
the at least one holding device (230a) of the further shell (230), that is mounted at a further fixing point (202f), is prevented by a blocking device (230b) of a next shell (230) from release from the further fixing point (202f) and so forth until a holding device (230a) of the last shell is mounted at a fixing point (202f) of the housing (202), that is prevented by a blocking device (230b) of the first shell (230) from release from the fixing point (202f), whereby
the shells (230) are non-releasably connected to the housing (202).

2. An injection device (200) according to claim 1 wherein the injection device (200) includes precisely two shells (230).

3. An injection device (200) according to claim 1 or claim 2 wherein the shells (230) are geometrically identical.

4. An injection device (200) according to claim 1 or claim 2 wherein the shell segments are identical.

5. An injection device (200) according to one of the preceding claims **characterised in that** the holding devices (230a) are snap arms with a tooth-like structure at the free end and the fixing points (202f) are recesses of a complementary configuration to the tooth-like structure.

6. An injection device (200) according to one of claims 1 to 4 **characterised in that** the holding devices (230a) are arms which have an opening at their free end and the fixing points (202f) include tooth-like structures, wherein the holding devices (230a) can come into snapping engagement with the fixing points (202f).

7. An injection device (200) according to claim 1 wherein the injection device (200) includes precisely three shells (230).

8. An injection device (200) according to claim 1 wherein the injection device (200) includes precisely four shells (230).

9. An injection device (200) according to claim 1 wherein the housing (202) includes at least one opening (202h) which gives a view on to a product container (13) containing the substance to be administered and at least one housing-like shell (230) mounted to the housing (202) in the region of the housing opening also includes an opening (230c), **characterised in that** the opening (230c) in the housing-like shell (230) comes to lie in the region of the housing opening when the shell (230) is mounted to the housing (202) so that the product container (13) remains at least partially visible even when the shell (230) is fitted.

10. An injection device (200) according to the preceding claim **characterised in that** the opening (202c) in the housing-like shell (230) is smaller than the opening (202h) in the housing (202).

11. An injection device (200) according to claim 1 **characterised in that** the fixing points (202f) have optically or electrically conductive regions.

12. An injection device (200) according to the preceding claim **characterised in that** the at least one holding device has optically or electrically conductive elements which can contact the optically or electrically conductive regions respectively of the fixing points (202f).

## Revendications

1. Dispositif d'injection (200) pour l'administration d'une substance fluide comprenant au moins
un boîtier (202) qui présente sur son côté extérieur un agencement de plusieurs points de fixation (202f),
le dispositif d'injection (200) comprenant en outre
au moins deux coques de type boîtier (230) au niveau desquelles respectivement
au moins un dispositif de retenue (230a) et
au moins un dispositif de blocage (230b) sont agencés,
dans lequel les coques (230) peuvent être montées sur le boîtier (202), **caractérisé en ce que** le dispositif de retenue (230a) est agencé de manière géométrique de sorte qu'il vienne se poser lors du montage de la coque (230) contre le boîtier (202) dans un point de fixation (202f) et contribue ainsi à la fixation de la coque (230) au niveau du boîtier (202) et dans lequel
l'au moins un dispositif de retenue (230a) et le dispositif de blocage (230b) sont agencés au niveau des coques (230) de sorte que
au moins un dispositif de retenue (230a) d'une première coque (230) puisse être monté au niveau du point de fixation (202f),
au moins un dispositif de retenue (230a) d'une seconde coque (230) puisse être monté au niveau d'un autre point de fixation (202f),
l'au moins un dispositif de retenue (230a) monté au niveau d'un point de fixation (202f) de la première coque (230) est empêché de se détacher du point de fixation (202f) par un dispositif de blocage (230b) de la seconde coque (230),
l'au moins un dispositif de retenue (230a) monté au niveau d'un autre point de fixation (202f) de la seconde coque (230) est empêché de se détacher de l'autre point de fixation (202f) par un dispositif de blocage (230b) d'une autre coque (230) ou, si le dispositif d'injection (200) comporte seulement deux coques (230), par un dispositif de blocage (230b) de la première coque (230), et
l'au moins un dispositif de retenue (230a) monté au niveau d'un autre point de fixation (202f) de l'autre coque (230) est empêché de se détacher de l'autre point de fixation (202f) par un dispositif de blocage (230b) d'une prochaine coque (230) et ainsi de suite jusqu'à ce qu'un dispositif de retenue (230a) de la dernière coque soit monté au niveau d'un point de fixation (202f) du boîtier (202) qui est empêché de se détacher du point de fixation (202f) par un dispositif de blocage (230b) de la première coque (230), par l'intermédiaire duquel
les coques (230) sont reliées de manière non détachable au boîtier (202).

2. Dispositif d'injection (200) selon la revendication 1, le dispositif d'injection (200) comporte précisément deux coques (230).

3. Dispositif d'injection (200) selon la revendication 1 ou 2, dans lequel les coques (230) sont identiques géométriquement.

4. Dispositif d'injection (200) selon la revendication 1 ou 2, dans lequel les segments de coque sont identiques.

5. Dispositif d'injection (200) selon l'une des revendications précédentes, **caractérisé en ce qu'**il s'agit pour les dispositifs de retenue (230a) de bras d'encliquetage avec une structure dentée à l'extrémité libre et pour les points de fixation (202f) de cavités réalisées de manière complémentaire à la structure dentée.

6. Dispositif d'injection (200) selon l'une des revendications 1 à 4, **caractérisé en ce qu'**il s'agit pour les dispositifs de retenue (230a) de bras qui présentent à leur extrémité libre une ouverture et les points de fixation (202f) comprennent des structures dentées, dans lequel les dispositifs de retenue (230a) peuvent s'encliqueter avec les points de fixation (202f).

7. Dispositif d'injection (200) selon la revendication 1, le dispositif d'injection (200) comporte précisément trois coques (230).

8. Dispositif d'injection (200) selon la revendication 1, le dispositif d'injection (200) comporte précisément quatre coques (230).

9. Dispositif d'injection (200) selon la revendication 1, dans lequel le boîtier (202) comporte au moins une ouverture (202h) qui libère la vue sur un récipient de produit (13) qui contient la substance à administrer et au moins une coque de type boîtier (230) qui est montée dans la zone de l'ouverture de boîtier au niveau du boîtier (202), comporte aussi une ouverture (230c), **caractérisé en ce que** l'ouverture (230c) vient se poser dans la coque (230) de type boîtier dans la zone de l'ouverture de boîtier lorsque la coque (230) est montée au niveau du boîtier (202) de sorte que le récipient de produit (13) reste aussi au moins partiellement visible en cas de coque montée (230).

10. Dispositif d'injection (200) selon la revendication précédente, **caractérisé en ce que** l'ouverture (202c) dans la coque de type boîtier (230) est inférieure à l'ouverture (202h) dans le boîtier (202).

11. Dispositif d'injection (200) selon la revendication 1, **caractérisé en ce que** les points de fixation (202f) présentent des zones conductrices optiquement ou électriquement.

12. Dispositif d'injection (200) selon la revendication précédente, **caractérisé en ce qu'**au moins un dispositif de retenue présente des éléments conducteurs optiquement ou électriquement qui peuvent toucher les zones conductrices optiquement ou électriquement des points de fixation (202f).
